# EUROPEAN PATENT APPLICATION

(11) **EP 1 669 089 A1**
(43) Date of publication of application: **14.06.2006**
(21) Application number: 04788406.9
(22) Date of filing: 30.09.2004
(51) Int. Cl.: A61K 45/00, A61P 9/10, A61P 21/00, A61P 25/16, A61P 25/28, C12Q 1/37, C12N 15/12, C12N 15/57, G01N 33/15, G01N 33/50

(54) **INHIBITION OF NERVE CELL DEATH BY INHIBITING DEGRADATION OF SHC3, ATF6 OR CREBL1 BY HtrA2 AND METHOD OF AMELIORATING NEURODEGENERATIVE DISEASES**

(30) Priority: 30.09.2003 JP 2003342588
(71) Applicant: DAIICHI PHARMACEUTICAL CO., LTD., Chuo-ku, Tokyo 103-8234 (JP)
(72) Inventor: DOI, Hirofumi, Makuhari Techno Garden D17, Chiba-shi, 261-8501 (JP); SAITO, Ken, Daiichi Pharmaceutical Co., Ltd., Tokyo 134-8630 (JP)
(74) Representative: De Clercq, Ann G. Y.
(86) International application number: PCT/JP2004/014378
(87) International publication number: WO 2005/030256

(57) **Abstract**

In the present invention, CREBL1 and SHC1 were found to interact with HtrA2, and it was revealed for the first time that that CREBL1, ATF6 that is a family of CREBL1, and SHC3 that is a family of SHC1 are degraded by active HtrA2.

The present invention provides a means for inhibiting neural cell death comprising inhibiting the degradation by HtrA2 of at least one of CREBL1, ATF6, and SHC3; a means for preventing, treating, or controlling diseases accompanied with neural cell death (for example, neurodegenerative diseases or brain ischemia), comprising inhibiting the aforementioned degradation: a method of identifying a compound that inhibits the degradation by HtrA2 of CREBL1 and/or ATF6; and a reagent kit.

## Description

### TECHNICAL FIELD

The present invention relates to inhibition of the degradation by HtrA2 of at least one of SHC3 (src homology 2 domain containing transforming protein C3), ATF6 (activating transcription factor 6), and CREBL1 (cAMP responsive element binding protein-like 1). More concretely, the present invention relates to a method for inhibiting neural cell death, comprising inhibiting the degradation by HtrA2 of at least one of SHC3, ATF6 and CREBL1, or a method for inhibiting neural cell death, comprising using one or more compounds that inhibit the degradation. Further, the present invention relates to a method for preventing, treating or controlling brain ischemia or neurodegenerative disease, comprising inhibiting the degradation by HtrA2 of at least one of SHC3, ATF6 and CREBL1. Still further, the present invention relates to a method of identifying a compound that inhibits the degradation by HtrA2 of at least one of SHC3, ATF6 and CREBL1. Further, the present invention relates to an agent for inhibiting neural cell death, comprising one or more compounds that inhibit the degradation by HtrA2 of at least one of SHC3, ATF6 and CREBL1. Still further, the present invention relates to an agent for preventing, treating or controlling brain ischemia or neurodegenerative disease, comprising one or more compounds that inhibit the degradation by HtrA2 of at least one of SHC3, ATF6 and CREBL1. Further, the present invention relates to a reagent kit, comprising at least one selected from the group consisting of HtrA2, a polynucleotide encoding HtrA2, and a vector containing the polynucleotide encoding HtrA2; and at least one selected from the group consisting of SHC3, ATF6, CREBL1, a polynucleotide encoding at least one of SHC3, ATF6 and CREBL1, and a vector containing the polynucleotide encoding at least one of SHC3, ATF6 and CREBL1.

### BACKGROUND ART

Organisms are taking self defense against stresses formed on the environmentals, by regulating the survival and apoptosis (cell death) of cells, but the disturbed regulation of the survival and apoptosis-controlling mechanism may cause excessive cell death to induce various disorders (Non-Patent Reference 1).

In recent years, researches have been remarkable progressed on mitochondria and endoplasmic reticulum as sites for perceiving and regulating stresses. It has been reported that HtrA2 protein (hereinafter referred to as HtrA2) located at a mitochondrial membrane translocates from the mitochondrial membrane to cytoplasm due to stress such as UV and heat shock (Non-Patent References 2-5), thereby induces two types of cell death, that is, caspase dependent cell death and caspase independent cell death (Non-Patent Reference 6). HtrA2 precursor protein (SEQ ID NO: 2) converts to the mature type (SEQ ID NO: 4) by being cleaved the N-tem-iinal 133 amino acids thereof and then translocates from mitochondria to cytoplasm. The mature HtrA2 has a protease activity. Herein after, HtrA2 with the protease activity may be referred to as active HtrA2.

It is known that caspase independent cell death is depending on the feature of HtrA2 itself as a serine protease and that caspase independent cell death is not triggered by the HtrA2 mutant with 306^{th} serine in the amino acid sequence of the precursor protein thereof (this position is corresponding to 174^{th} in mature type) substituted with alanine (Non-Patent References 3-5). Such HtrA2 mutant has no protease activity. Herein after, HtrA2 without the protease activity may be referred to as inactive HtrA2. As mentioned, HtrA2 is a factor that plays important roles in the mechanism of inducing caspase independent cell death.

The References cited in the specification are listed as follows:
Non-patent Reference 1: "Saibou Kougaku (Cell Technology) ", 2002, Vol.21, No.4, p.360-394.
Non-patent Reference 2: Yamaguchi H. et al., "Cancer Research", 2003, Vol.63, p.1483-1489.
Non-patent Reference 3: Suzuld Y. et al., "Molecular Cell", 2001, Vol.8, p.613-621.
Non-patent Reference 4: Hegde R.et al., "The Journal of Biological Chemistry", 2002, Vol.277, p.432-438.
Non-patent Reference 5: Martins L.M. et al., "The Journal of Biological Chemistry", 2002, Vol.277, p.439-444.
Non-patent Reference 6: "Jikken Igaku (Experimental Medicine) ", 2002, Vol.20, No.1, p.73-75.
Non-patent Reference7: Conti L. et al., "Nature Neuroscience", 2001, Vol.4, p.579-586.
Non-patent Reference8: "Seikagaku (Biochemistry) ", 2001, Vol.73, No. 11, p.1322-1325.
Non-patent Reference9: Kaufinan R.J. et al., "The Journal of Clinical Investigation", 2002, Vol.110, p.1389-1398.
Non-patent Reference 10: Haze K. et al., "The Biochemical Journal", 2001, Vol.355, p.19-28.
Non-patent Reference 11: Ulmer K.M., "Science", 1983, Vol.219, p.666-671.
Non-patent Reference 12: "PEPUTIDO GOUSEI", Maruzen Co., Ltd., 1975.
Non-patent Reference 13: "Peptide Synthesis", Interscience, New York, 1996.

### DISCLOSURE OF THE INVENTION

### [PROBLEM TO BE SOLVED BY THE INVENTION]

Today, caspase independent cell death in neurodegenerative diseases has been reported in many papers. Meanwhile, it was reported that neural cell death is caused through endoplasmic reticulum stress in brain ischemia. HtrA2 has been considered to play an important role in the mechanism for inducing caspase independent cell death. From these facts, it is believed that HtrA2 is likely to be a new target of neurodegenerative disease and that inhibition of the neural cell death due to endoplasmic reticulum stress may become a novel target for drug discovery.

Under the circumstances where a mechanism of caspase independent cell death caused by HtrA2 and a substrate of HtrA2 have not yet been clarified, an object of the present invention is to find out a protein that interacts with HtrA2 and to provide a means that allows prevention and/or treatment of disorders attributable to the degradation of the protein by HtrA2.

### [MEANS FOR SOLVING THE PROBLEM]

The present inventors have concentrated intensively efforts to solve the aforementioned problem. We predicted *in-silico* that HtrA2 interacts with CREBL1 and SHC1, and then found experimentally that CREBL1, ATF6 that is a family member of CREBL1, and SHC3 that is a family member of SHC1, were degraded by active HtrA2. Thus, we achieved the present invention.

Namely, the present invention relates to a method for inhibiting neural cell death, comprising inhibiting the degradation by HtrA2 (high temperature requirement protein A2) of at least one of SHC3 (src homology 2 domain containing transforming protein C3), ATF6 (activating transcription factor 6), and CREBL1 (cAMP responsive element binding protein-like 1).

The present invention also relates to a method for inhibiting neural cell death, comprising using one or more compounds that inhibit the degradation by HtrA2 of at least one of SHC3, ATF6 andCREBL1.

The present invention further relates to the aforementioned method for inhibiting neural cell death, in which the neural cell death is neural cell death attributable to brain ischemia.

The present invention still further relates to a method for preventing, treating or controlling brain ischemia or neurodegenerative disease, comprising inhibiting the degradation by HtrA2 of at least one of SHC3, ATF6 and CREBL1.

The present invention also relates to the aforementioned preventing, treating, or controlling method, in which the neurodegenerative disease is Alzheimer's disease, Parkinson's disease, polyglutamine disease, prion disease, or amyotrophic lateral sclerosis.

The present invention further relates to a method of identifying a compound that inhibits the degradation by HtrA2 of at least one of SHC3, ATF6 and CREBL1, comprising contacting HtrA2 and at least one of SHC3, ATF6 and CREBL1 with a compound under conditions that allow the degradation by HtrA2 of at least one of SHC3, ATF6 and CREBL1; introducing a system using a signal and a marker capable of detecting at least one of SHC3, ATF6 and CREBL1; detecting the presence or absence and/or change of the signal and the marker; and determining whether the compound inhibits the degradation of at least one of SHC3, ATF6 and CREBL1.

The present invention still further relates to a method of identifying a compound that inhibits the degradation by HtrA2 of at least one of SHC3, ATF6 and CREBL1, comprising contacting HtrA2 and at least one of SHC3, ATF6 and CREBL1 with a compound under conditions that allow the degradation by HtrA2 of at least one of SHC3, ATF6 and CREBL1; detecting the presence or absence of at least one of SHC3, ATF6 and CREBL1, and/or measuring the change of the amount thereof; or detecting the presence or absence of the degradation product of at least one of SHC3, ATF6 and CREBL1, and/or measuring the change of the amount thereof ; and determining whether the compound inhibits the degradation of at least one of SHC3, ATF6 and CREBL1.

The present invention also relates to an agent for inhibiting neural cell death, comprising one or more compounds that inhibit the degradation by HtrA2 of at least one of SHC3, ATF6 and CREBL1.

The present invention further relates to the aforementioned agent for inhibiting neural cell death, wherein the neural cell death is neural cell death attributable to brain ischemia.

The present invention still further relates to an agent for preventing, treating or controlling brain ischemia or neurodegenerative disease, comprising one or more compounds that inhibit the degradation by HtrA2 of at least one of SHC3, ATF6 and CREBL1.

The present invention also relates to the aforementioned agent for preventing, treating or controlling neurodegenerative disease, wherein the neurodegenerative disease is Alzheimer's disease, Parkinson's disease, polyglutamine disease, prion disease, or amyotrophic lateral sclerosis.

The present invention further relates to a reagent kit, comprising at least one selected from the group consisting of HtrA2, a polynucleotide encoding HtrA2, and a vector containing the polynucleotide encoding HtrA2; and at least one selected from the group consisting of SHC3, ATF6, CREBL1, a polynucleotide encoding at least one of SHC3, ATF6 and CREBL1, and a vector containing the polynucleotide encoding at least one of SHC3, ATF6 and CREBL1.

### [ADVANTAGE OF THE INVENTION]

According to the present invention, to inhibit the degradation by HtrA2 of at least one of CREBL1, ATF6, and SHC3 enables to inhibit the reduction or disappearance of the amount of at least one of these proteins, and as result, enables to inhibit cell death (for example, neural cell death) and to prevent, treat, and control diseases accompanied with cell death (such as brain ischemia and neurodegenerative diseases).

SHC3 is expressed specifically in mature central nerve cells and is considered to play an important role in the differentiation and survival of neural cells (Non-Patent References 7 and 8). ATF6 and CREBL1 are considered to perceive the endoplasmic reticulum stress to induce transcription of chaperon genes and involve in the recovery from the endoplasmic reticulum stress in cells and survival of the cells (Non-Patent References 9 and 10). From these facts, it is believed that the degradation by HtrA2 of at least SHC3, ATF6, and CREBL1 causes the reduction or disappearance of the amount of these proteins, and thereby induces the enhanced cell death due to stress and the like, such as neural cell death. Therefore, to inhibit the degradation by HtrA2 of at least SHC3, ATF6, and CREBL1 and thereby to inhibit the reduction or disappearance of the amount of these proteins enable to inhibit cell death (such as neural cell death), and enable to prevent, treat, or control of diseases accompanied with cell death (for example, brain ischemia or neurodegenerative diseases).

According to the present invention, a method of identifying a compound that inhibits the degradation by HtrA2 of at least one of SHC3, ATF6 and CREBL1 can be also carried out. The compound obtained by the identification method can be used for inhibiting cell death (for example, neural cell death), diabetes, and for preventing, treating, or controlling a disease accompanied with cell death (such as brain ischemia and neurodegenerative diseases).

### BRIEF DESCRIPTION OF THE DRAWINGS

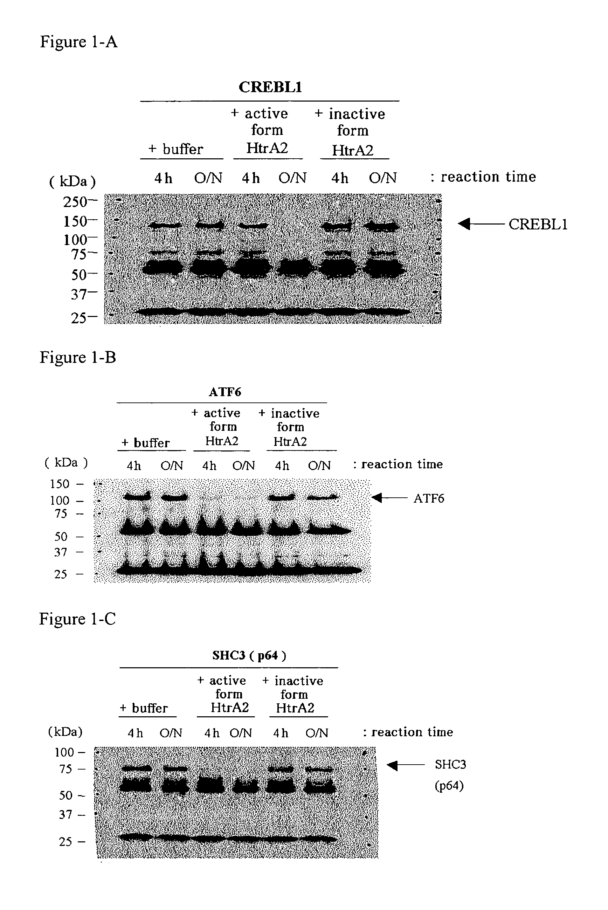

Fig. 1-A shows that CREBL1 was degraded in vitro by active HtrA2 (mature HtrA2). The band of CREBL1 was significantly reduced with the reaction of active HtrA2 with CREBL1 for overnight (O/N). On the other hand, the degradation of CREBL1 by inactive HtrA2 (mature HtrA2 (S306A)) was not observed (Example 2).

Fig. 1-B shows that ATF6 was degraded in vitro by active HtrA2 (mature HtrA2). The band of ATF6 was significantly reduced with the reaction of active HtrA2 with ATF6 for 4 hours (4h) or for overnight (O/N). On the other hand, the degradation of ATF6 by inactive HtrA2 (mature HtrA2 (S306A)) was not observed (Example 2).

Fig. 1-C shows that SHC3 (p64) was degraded in vitro by active HtrA2 (mature HtrA2). The band of SHC3 (p64) was significantly reduced with the reaction of active HtrA2 with SHC3 (p64) for four hours (4h) or overnight (O/N). On the other hand, the degradation of SHC3 (p64) by inactive HtrA2 (mature HtrA2 (S306A)) was not observed (Example 2).

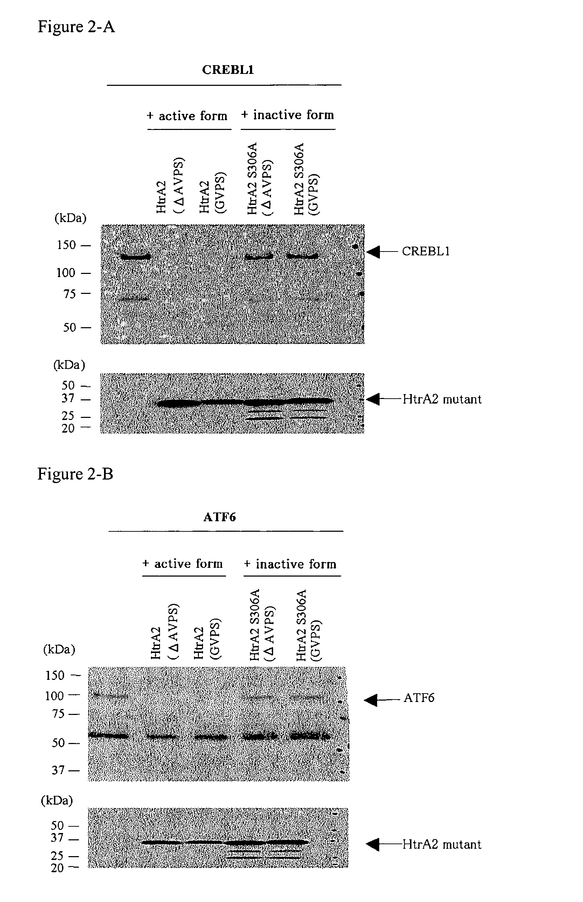

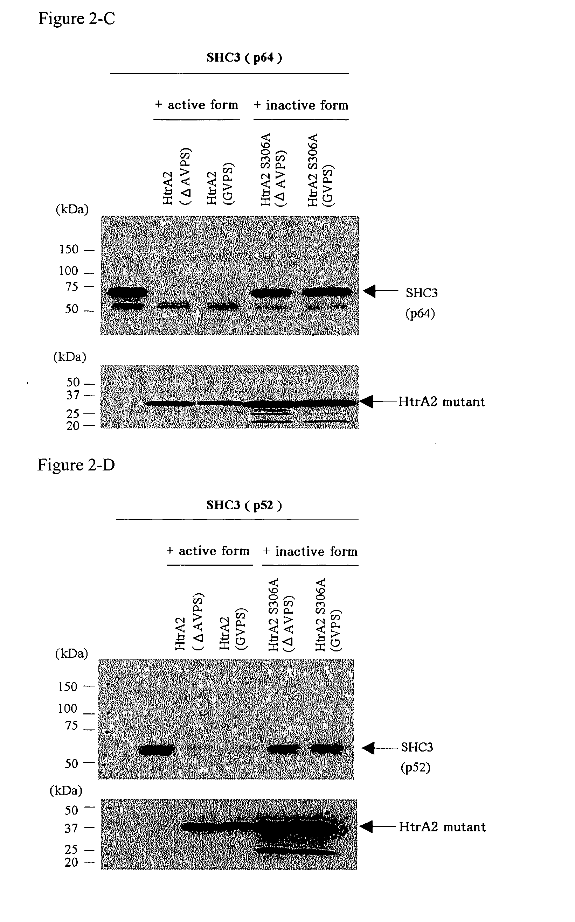

Fig. 2-A shows that CREBL1 was degraded in a cell by active HtrA2 mutant (mature HtrA2 (ΔAVPS) that lacks N-terminal four amino acid residues of the active HtrA2 (mature HtrA2); or mature HtrA2 (GVPS) with the substitution of alanine at the N-terminal amino acid of the active HtrA2 with glycine) (upper panel). In the analysis using cells in which the mature HtrA2 (ΔAVPS) or the mature HtrA2 (GVPS) was co-expressed with CREBL1, the band of CREBL1 was significantly reduced (upper panel). On the other hand, in the analysis using cells in which inactive HtrA2 mutant (the mature HtrA2 S306 (ΔAVPS) or the mature HtrA2 S306 (GVPS)) was co-expressed with CREBL1, reduction of the band of CREBL1 was not observed (upper panel). The expression of each HtrA2 mutant in the cells was almost same among the cells (lower panel) (Example 3).

Fig. 2-B shows that ATF6 was degraded in a cell by active HtrA2 mutant (mature HtrA2 (ΔAVPS) that lacks N-terminal four amino acid residues of the active HtrA2 (mature HtrA2); or mature HtrA2 (GVPS) with the substitution of alanine at the N-terminal amino acid of the active HtrA2 with glycine) (upper panel). In the analysis using cells in which the mature HtrA2 (ΔAVPS) or the mature HtrA2 (GVPS) was co-expressed with ATF6, the band of ATF6 was significantly reduced (upper panel). On the other hand, in the analysis using cells in which inactive HtrA2 mutant (the mature HtrA2 S306 (ΔAVPS) or the mature HtrA2 S306 (GVPS)) was co-expressed with ATF6, reduction of the band of ATF6 was not observed (upper panel). The expression of each HtrA2 mutant in the cells was almost same among the cells (lower panel) (Example 3)

Fig. 2-C shows that SHC3 (p64) was degraded in a cell by active HtrA2 mutant (mature HtrA2 (ΔAVPS) that lacks N-terminal four amino acid residues of the active HtrA2 (mature HtrA2); or mature HtrA2 (GVPS) with the substitution of alanine at the N-terminal amino acid of the active HtrA2 with glycine) (upper panel). In the analysis using cells in which the mature HtrA2 (ΔAVPS) or the mature HtrA2 (GVPS) was co-expressed with SHC3 (p64), the band of SHC3 (p64) was significantly reduced (upper panel). On the other hand, in the analysis using cells in which inactive HtrA2 mutant (the mature HtrA2 S306 (ΔAVPS) or the mature HtrA2 S306 (GVPS)) was co-expressed with SHC3 (p64), reduction of the band of SHC3 (p64) was not observed (upper panel). The expression of each HtrA2 mutant in the cells was almost same among the cells (lower panel) (Example 3)

Fig. 2-C shows that SHC3 (p52) was degraded in a cell by active HtrA2 mutant (mature HtrA2 (ΔAVPS) that lacks N-terminal four amino acid residues of the active HtrA2 (mature HtrA2); or mature HtrA2 (GVPS) with the substitution of alanine at the N-terminal amino acid of the active HtrA2 with glycine) (upper panel). In the analysis using cells in which the mature HtrA2 (ΔAVPS) or the mature HtrA2 (GVPS) was co-expressed with SHC3 (p52), the band of SHC3 (p52) was significantly reduced (upper panel). On the other hand, in the analysis using cells in which inactive HtrA2 mutant (the mature HtrA2 S306 (ΔAVPS) or the mature HtrA2 S306 (GVPS)) was co-expressed with SHC3 (p52), reduction of the band of SHC3 (p52) was not observed (upper panel). The expression of each HtrA2 mutant in the cells was almost same among the cells (lower panel) (Example 3)

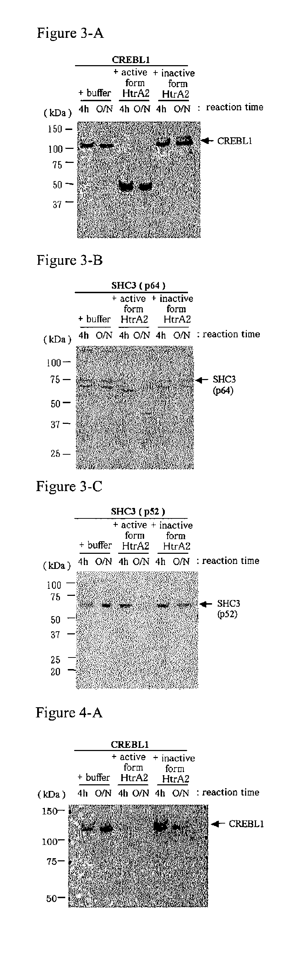

Fig. 3-A shows that the degradation of CREBL1 by active HtrA2 (mature HtrA2) was detected in the study using N-temlinal tagged-CREBL1 with biotinylated lysine residue within the protein and detecting the biotin. The band of biotinylated CREBL1 was significantly reduced with the reaction of active HtrA2 with CREBL1 for 4 hours (4h) or overnight (O/N), and the band around 50 kDa was detected which is considered to be a band indicating the degradation product of CREBL1. On the other hand, the degradation of CREBL1 by inactive HtrA2 (mature HtrA2 (S306A)) was not observed, and the band around 50kDa as above was not detected (Example 4).

Fig. 3-B shows that the degradation of SHC3 (p64) by active HtrA2 (mature HtrA2) was detected in the study using N-terminal tagged- SHC3 (p64) with biotinylated lysine residue within the protein and detecting the biotin. The band of biotinylated SHC3 (p64) was significantly reduced with the reaction of active HtrA2 with SHC3 (p64) for 4 hours (4h) or overnight (O/N), and the bands around 70 kDa, 40kDa and 30kDa were detected which are considered to be the bands indicating the degradation products of SHC3 (p64). On the other hand, the degradation of SHC3 (p64) by inactive HtrA2 (mature HtrA2 (S306A)) was not observed, and the bands around 70 kDa, 40kDa and 30kDa as above were not detected (Example 4).

Fig. 3-C shows that the degradation of SHC3 (p52) by active HtrA2 (mature HtrA2) was detected in the study using N-terminal tagged- SHC3 (p52) with biotinylated lysine residue within the protein and detecting the biotin. The band of biotinylated SHC3 (p52) was significantly reduced with the reaction of active HtrA2 with SHC3 (p52) overnight (O/N), but a band that is considered to be the bands indicating a degradation product of SHC3 (p52) was not detected. On the other hand, the degradation of SHC3 (p52) by inactive HtrA2 (mature HtrA2 (S306A)) was not observed (Example 4).

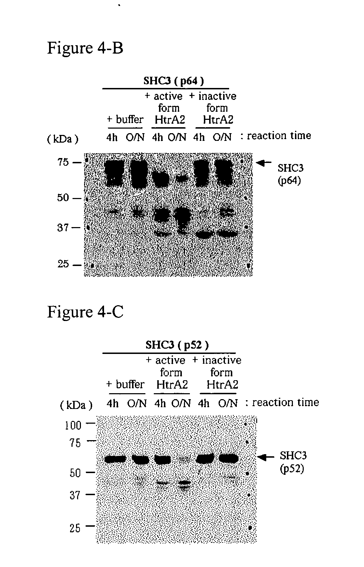

Fig. 4-A shows that the degradation of CREBL1 by active HtrA2 (mature HtrA2) was detected in the study using N-terminal tagged-CREBL1 with biotinylated lysine residue within the protein and detecting the Tag ligated at the N-terminal. The N-terminal tagged-CREBL1 was significantly reduced with the reaction of active HtrA2 with CREBL1 for 4 hours (4h) or overnight (O/N). The band around 50 kDa that was detected in the study shown in Fig. 3, which is considered to be a band indicating the degradation product of CREBL1, was not detected using anti-Tag antibody. On the other hand, the degradation of CREBL1 by inactive HtrA2 (mature HtrA2 (S306A)) was not observed (Example 4).

Fig. 4-B shows that the degradation of SHC3 (p64) by active HtrA2 (mature HtrA2) was detected in the study using C-terminal tagged-SHC3 (p64) with biotinylated lysine residue within the protein by detecting the Tag ligated at the C-terminal. The band of C-terminal tagged-SHC3 (p64) was significantly reduced with the reaction of active HtrA2 with SHC3 (p64) for 4 hours (4h) or overnight (O/N), and the bands around 70 kDa, 40kDa, and 30kDa which are considered to be a band indicating the degradation products of SHC3 (p64), were detected. On the other hand, the degradation of SHC3 (p64) by inactive HtrA2 (mature HtrA2 (S306A)) was not observed (Example 4).

Fig. 4-C shows that the degradation of SHC3 (p52) by active HtrA2 (mature HtrA2) was detected in the study using C-terminal tagged-SHC3 (p52) with biotinylated lysine residue within the protein by detecting the Tag ligated at the C-terminal. The band of C-terminal tagged-SHC3 (p52) was significantly reduced with the reaction of active HtrA2 with SHC3 (p52) overnight (O/N), and the band around 40kDa which is considered to be a band indicating the degradation product of SHC3 (p52), was detected. On the other hand, the degradation of SHC3 (p52) by inactive HtrA2 (mature HtrA2 (S306A)) was not observed (Example 4).

### DETAILED DESCRIPTION OF THE INVENTION

The present invention claims the benefit of priority from Japanese Patent Application No. 2003-342588, which is incorporated herein by reference in its entirety.

In the present specification, the term "polypeptide" may be used as a generic term which includes the followings: an isolated or a synthetic full-length protein; an isolated or a synthetic full-length polypeptide; and an isolated or a synthetic full-length oligopeptide. A protein, a polypeptide or an oligopeptide used herein comprises two or more amino acids that are bound to each other by peptide bond or modified peptide bond. Herein after, an amino acid may be represented by a single letter or by three letters.

In the present invention, the interaction of HtrA2 with CREBL1 and with SHC1 was predicted *in-silico* according to the method described in the pamphlet of Intemational Publication No. WO 01/67299. Further, it was revealed by way of experiment, for the first time, that HtrA2 degrades CREBL1, ATF6 belonging to the CREBL1 family and SHC3 belonging to the SHC1 family.

SHC3 is also referred to as N-Shc or ShcC and is specifically expressed in the mature central nerve cells. SHC3 is considered to transmit a signal from Trk family, a receptor tyrosine kinase, activated by the neurotrophic factors (such as NGF and EGF) and to play an important role in the differentiation and survival of neural cells via PI3K-Akt-Bad pathway. In addition, it has been also reported that cells in which only SH domain of SHC3 (dominant negative) was expressed exhibited cell death and showed suppressed neurite extension (Non-Patent References 7 and 8).

CREBL1 is one of ATF6 family and is also referred to as ATF6*β*. It has been known that locates in endoplasmic reticulum and the portion thereof translocates into nucleus upon endoplasmic reticulum stress after processing by S1P and S2P to work as a transcription factor.

It has been known that both CREBL1 and ATF6 perceive the endoplasmic reticulum stress to induce transcription of chaperon genes, and involve in the recovery of the stress in cells and the survival of the cells (Non-Patent References 9 and 10). In addition, there are some reports demonstrating the relation between endoplasmic reticulum stress and neurodegenerative diseases (Non-Patent Reference 1).

From these facts, it is believed that the degradation by HtrA2 of at least one of SHC3, ATF6, and CREBL1 causes the reduction or disappearance of the amount of these proteins, and thereby induces the disruption of signal pathway for the development and survival of neural cells, resulting in the enhanced neural cell death.

Accordingly, to inhibit the degradation by HtrA2 of at least one of SHC3, ATF6, and CREBL1 enables to inhibit cell death. Further to inhibit the degradation by HtrA2 of at least one of SHC3, ATF6, and CREBL1 enables the prevention and/or treatment of diseases accompanied with cell death. For example, to inhibit the degradation by HtrA2 of at least one of SHC3, ATF6, and CREBL1 enables to inhibit neural cell death, such as the neural cell death due to endoplasmic reticulum stress. Furthermore, it can be conducted to prevent, treat, or control the diseases accompanied with cell death, for example, neurodegenerative diseases and brain ischemia. In addition, it is believed that an agent for inhibiting the degradation by HtrA2 of at least one of SHC3, ATF6, and CREBL1 may become a pharmaceutical agent for treating neurodegenerative diseases and brain ischemia.

Based on these findings thus obtained, the present invention provides a method for inhibiting the degradation by HtrA2 of at least one of SHC3, ATF6, and CREBL1. The present invention also provides a method for inhibiting neural cell death, comprising inhibiting the degradation by HtrA2 of at least one of SHC3, ATF6, and CREBL. The method for inhibiting neural cell death according to the present invention may be conducted preferably in vitro. Further, the present invention provides a method for preventing, treating or controlling diseases attributable to the degradation of at least one of SHC3, ATF6, and CREBL1.

A method for inhibiting the degradation of at least one of SHC3, ATF6, and CREBL1 can be carried out by inhibiting the function of HtrA2. For example, the method can be carried out by inhibiting the interaction of HtrA2 with SHC3, ATF6, or CREBL1. Alternatively, the method can be carried out by inhibiting the enzyme activity of HtrA2. The inhibition of the degradation of at least one of SHC3, ATF6, and CREBL1 can be achieved by inhibiting the function of HtrA2 preferably in an in vitro sample comprising HtrA2 and at least one of SHC3, ATF6, and CREBL1. Herein, such a substance that has an inhibitory effect (for example, a peptide, an antibody and a low molecular weight compound described later that have a competitive inhibitory effect) is referred to "an inhibitor". "Interaction of HtrA2 with SHC3, ATF6, or CREBL1" as described herein means that HtrA2 and SHC3, ATF6, or CREBL1 affect each other in a certain manner, and as a specifically result, HtrA2 may degrade SHC3, ATF6, or CREBL1. The term "in a certain manner" includes "by binding to", "by contacting with", or "by being adjacent to", and may be any manner as long as it allows their affection each other.

An inhibition of the degradation by HtrA2 of at least one of SHC3, ATF6, and CREBL1 can be carried out using a compound that inhibits the interaction of at least one of SHC3, ATF6, and CREBL1 with HtrA2. Such a compound may be, for example, a polypeptide comprising the amino acid sequence of a site, in each of amino acid sequences of SHC3, ATF6, CREBL1 or HtrA2, where SHC3, ATF6, or CREBL1 interacts with HtrA2. In particular, such a polypeptide derived from SHC3, ATF6, or CREBL1 which serves as the substrate of HtrA2 can competitively inhibit the interaction of SHC3, ATF6, or CREBL1, with HtrA2, and can inhibit the degradation by HtrA2 of these proteins. Such a polypeptide can be obtained by designing polypeptides based on the amino acid sequence of HtrA2, SHC3, ATF6, or CREBL1, synthesizing them by peptide synthesis methods well-known in the art, and selecting a polypeptide that inhibits the degradation of at least one of SHC3, ATF6, or CREBL1 by HtrA2. For example, the polypeptide comprising the amino acid sequence of a degradation site by HtrA2 in each of amino acid sequences of SHC3, ATF6, or CREBL1 may be preferably used for such a compound. A polypeptide having an amino acid sequence derived from the thus specified polypeptide, in which a mutation such as a deletion, substitution, addition or insertion of one to several amino acids was introduced, is also included in the scope of the present invention. A polypeptide that inhibits the degradation by HtrA2 of SHC3, ATF6, or CREBL1, is preferable for such a polypeptide into which the mutation is introduced. A polypeptide having the mutation may be a naturally existing polypeptide or a polypeptide in which a mutation was introduced. Techniques for introducing a mutation such as a deletion, substitution, addition or insertion are known. For example, the Ulmer technique (Non-patent Reference 11) may be utilized. When introducing a mutation as described above, in view of avoiding a change in the fundamental properties (such as physical properties, function, physiological activity, and immunological activity) of the polypeptide, mutual substitution among homologous amino acids (polar amino acids, non-polar amino acids, hydrophobic amino acids, hydrophilic amino acids, positive-charged amino acids, negative-charged amino acids and aromatic amino acids or the like) may be readily conceived. Furthermore, these usable polypeptides can be modified to the extent that no significant functional change is involved, such as modification of its constituent amino group or carboxyl group and the like by an amidation and the like.

The aforementioned polypeptide can be produced by common methods that are known in peptide chemistry. A method described in the References (Non-patent documents 12 and 13), for example, may be used, although the methods are not limited thereto, and known methods can be broadly utilized. Particularly, a peptide synthesis method using an ordinary liquid phase method and solid phase method, such as, for example, the Fmoc method, can be used. Moreover, an amino acid synthesizer that is commercially available can be used for producing the polypeptide. Alternatively, a gene engineering technology can be also used for producing the polypeptide. For example, a gene encoding a polypeptide of interest can be used to prepare a recombinant expression vector that can express the gene in a host cell, followed by transfection of the recombinant expression vector into a suitable host cell such as E. coli to obtain a transformant. Culturing the transformant and collecting the polypeptide of interest from the obtained culture product achieves the production of the polypeptide.

An inhibition of the degradation by HtrA2 of SHC3, ATF6, or CREBL1 can be carried out using an antibody that recognizes HtrA2, SHC3, ATF6, or CREBL1 and inhibits the degradation by HtrA2 of SHC3, ATF6, or CREBL1. Such an antibody can be produced by known methods for preparing an antibody using HtrA2, SHC3, ATF6, or CREBL1, a polypeptide comprising the amino acid sequence of an interaction site of SHC3, ATF6, or CREBL1 with HtrA2, or the like as an antigen.

An inhibition of the degradation by HtrA2 of SHC3, ATF6, or CREBL1 can be carried out using a compound that inhibits the enzyme activity of HtrA2, preferably using a compound that specifically inhibits the enzyme activity of HtrA2. Such a compound can be obtained, for example, using at least one of SHC3, ATF6, and CREBL1 as a substrate by identifying a compound that inhibits the degradation of the substrate. "Specifically inhibit" denotes that "inhibit HtrA2 strongly, but does not inhibit or weakly inhibit other enzymes".

A method of identifying a compound that inhibits the degradation by HtrA2 of at least one of SHC3, ATF6, and CREBL1 can be constructed by utilizing a known pharmaceutical screening system. For example, a compound that inhibits the degradation by HtrA2 of at least one of SHC3, ATF6, and CREBL1 can be identified by selecting conditions that allow for the degradation by HtrA2 of at least one of SHC3, ATF6, and CREBL1, contacting a compound to be tested (hereinafter, referred to the test compound) with HtrA2 and/or at least one of SHC3, ATF6, and CREBL1 under the conditions, employing a system that uses a signal and/or a marker capable of detecting the degradation of at least one of SHC3, ATF6, and CREBL1, and then detecting the presence, the absence or the change of the signal and/or the marker. The condition for allowing the degradation by HtrA2 at least one of SHC3, ATF6, and CREBL1 may be a condition in vitro or in vivo. For example, a cell in which HtrA2 is coexpressed with at least one of SHC3, ATF6, and CREBL1 may be used. The co-expression in the cell can be accomplished by using an appropriate vector containing a polynucleotide encoding SHC3, ATF6, or CREBL1 and an appropriate vector containing a polynucleotide encoding HtrA2, and transfecting them into the cell by a conventional genetic engineering method. The vector containing a polynucleotide encoding SHC3, ATF6, or CREBL1 can be used solely or in combination thereof to transfect the cell. The cells may be, for example, a eukaryotic cell, preferably an animal cell, more preferably a cultured animal cell line, further preferably a cultured mammal cell line. Contact of the test compound with HtrA2 and/or at least one of SHC3, ATF6, and CREBL1 may be conducted prior to a degradation reaction of at least one of SHC3, ATF6, and CREBL1 by HtrA2, or may be conducted by allowing the test compound to coexist in the degradation reaction. As used herein, the term "marker" refers to a chemicals that can not be detected itself by physical properties or chemical propertied, but can generate the aforementioned signal via a chemical reaction and can be detected indirectly by using the physical properties or chemical properties of the generated signal as an indicator. As a signal and/or marker, the followings maybe used: a reporter gene (such as chloramphenicol acetyl transferase gene), radioisotope, tag peptides (such as His-tag, Myc-tag, HA-tag, FLAG-tag or Xpress-tag), enzymes such as GST, biotin and fluorescent protein. A signal and/or marker are not limited to these specific examples, and any labeling chemicals generally used in a method of identifying compounds may be utilized. Methods for detecting these signals or markers are known to those skilled in the art. As a convenient method, the degradation of at least one of SHC3, ATF6, and CREBL1 by HtrA2 can be detected by determining the presence, the absence and/or the change of the amount of these proteins or the amount of degradation products of these proteins. Determination of the amount of these proteins or the amount of degradation products of these proteins can be carried out using a known method for detecting a protein or peptide, such as, for example, Western blotting.

All of HtrA2, SHC3, ATF6, and CREBL1 are known proteins and disclosed in GenBank with the accession numbers NM_013247, NM_06848, NM_007348, and NM_00438, respectively.

The amino acid sequence of HtrA2 used in the Examples is shown in SEQ ID NO: 4. The nucleotide sequence of HtrA2 DNA encoding the amino acid sequence set forth in SEQ ID NO: 4 is shown in SEQ ID NO: 3. The polypeptide shown by the amino acid sequence set forth in SEQ ID NO:4 is a mature HtrA2. The mature HtrA2 denotes a mature protein that is generated from HtrA2 precursor protein (SEQ ID NO: 2) by cleavage of its N-terminal 133 amino acid residues and has a protease activity. Hereinafter, the HtrA2 with protease activity may be referred to as an active HtrA2. Further, a protein (SEQ ID NO: 8, which may be referred to as mature HtrA2 (ΔAVPS)) that lacks the N-terminal four amino acid residues (AVPS) in the mature HtrA2, or a protein (SEQ ID NO: 10, in some cases referred to as mature HtrA2 (GVPS)) with the substitution of alanine among the N-terminal four amino acid residues of the mature HtrA2 with glycine may be used as active HtrA2. Such HtrA2 with an introduced mutation can be used as active HtrA2 since there is no change in the protease activity. In the meantime, HtrA2 without protease activity may be in some cases referred to as inactive HtrA2. The inactive HtrA2 may be, for example, HtrA2 mutant without protease activity resulting from a mutation of the amino acid residue at the site necessary for protease activity of HtrA2 in the amino acid sequence of HtrA2. The site necessary for protease activity of HtrA2 includes a protease activity domain, more preferably the 174^{th} serine residue of mature HtrA2 (SEQ ID NO: 4) (which is corresponding to the 306^{th} residue of the precursor protein (SEQ ID NO: 2)). More specifically, the inactive HtrA2 can be, for example, HtrA2 mutant (SEQ ID NO: 6, which may be referred to as mature HtrA2 (S306A)) with a substitution of the 174^{th} serine residue of mature HtrA2 (which is corresponding to the 306^{th} residue of the precursor protein (SEQ ID NO: 2)) with alanine. Further, a protein (SEQ ID NO: 12, in some cases referred to as mature HtrA2 (S306A, ΔAVPS)) that lacks the N-terminal four amino acid residues (AVPS) in the mature HtrA2 (S306A), or a protein (SEQ ID NO: 14, in some cases referred to as mature HtrA2 (S306A, GVPS)) with the substitution of alanine among the N-terminal amino four amino acid residues of the mature HtrA2 with glycine may be used as inactive HtrA2.

The amino acid sequence of SHC3 used in the Examples is shown in SEQ ID NO: 16. Hereinafter, SHC3 protein shown by the amino acid sequence set for in SEQ ID NO: 16 may be sometimes referred to SHC3 (p64). The nucleotide sequence of SHC3 (p64) DNA is shown in SEQ ID NO: 15. It was found that the nucleotide sequence set for in SEQ ID NO: 15 had a difference in six nucleotides compared with the nucleotide sequence of SHC3 disclosed in accession number NM_01648 (see Example 2). In addition, SHC3 (p52) that is a splicing variant of SHC3 was used in the present invention. SHC3 (p52) is a protein that is translated from the second ATG (360 bases down stream from the first ATG) within the coding region of SHC3 gene.

The amino acid sequence of ATF6 used in the Examples is shown in SEQ ID NO: 20. In addition, the nucleotide sequence of ATF6 DNA is shown in SEQ ID NO: 19. It was found that the nucleotide sequence set forth in SEQ ID NO: 19 had a difference in fifteen nucleotides compared with the nucleotide sequence of ATF6 disclosed in accession number NM_007348 (see Example 2).

The amino acid sequence of CREBL1 used in the Examples is shown in SEQ ID NO: 18. Furthermore, the nucleotide sequence of CREBL1 DNA is shown in SEQ ID NO: 17. It was found that the nucleotide sequence set forth in SEQ ID NO: 17 had a difference in one nucleotide compared with the nucleotide sequence of CREBL1 disclosed in accession number NM_00438 (see Example 2).

In the present invention, HtrA2, SHC3, ATF6 and CREBL1, as well as each gene that encodes each of these proteins, are not limited to proteins or genes that are shown by the above-exemplified amino acid sequences or the above-exemplified nucleotide sequences, and includes generally reported HtrA2, SHC3, ATF6 and CREBL1.

HtrA2, SHC3, ATF6 and CREBL1 used in the present invention can be those prepared from cells in which these are expressed by means of genetic engineering techniques, or from any biological samples, or can be the products of cell-free synthesis systems or the chemical synthesis products. These can be subsequently further purified for use. Further, cells in which one or more of HtrA2, SHC3, ATF6 and CREBL1 are expressed by means of genetic engineering techniques can be used. Moreover, HtrA2, SHC3, ATF6 and CREBL1 can be labeled by ligating a different type of protein or polypeptide thereto at the N-terminus or the C-terminus, directly or indirectly via a linker peptide and the like, by means of, for example, genetic engineering techniques, as long as it has no influence upon the interaction of HtrA2 with SHC3, ATF6 or CREBL1, and upon the function of these proteins, such as protease activity of HtrA2, the feature of SHC3, ATF6 and CREBL1 as an enzyme substrate. A different type of protein or polypeptide may be, for example, β-galactosidase, an immunoglobulin Fc fragment (such as IgG) and a tag peptide (such as His-tag, Myc-tag, HA-tag, FLAG-tag, or Xpress-tag).

A polynucleotide encoding HtrA2, SHC3, ATF6 or CREBL1 can be prepared from a human cDNA library by known genetic engineering techniques. A vector containing the polynucleotide that encodes HtrA2, SHC3, ATF6 or CREBL1 can be obtained by introducing the polynucleotide using known genetic engineering techniques into a suitable expression vector, such as a vector derived from a bacterial plasmid.

A test compound may be, for example, a compound derived from a chemical library or natural products, as well as a compound obtained by drug design based on the primary structure or tertiary structure of HtrA2, SHC3, ATF6 or CREBL1. Alternatively, a compound obtained by drug design based on the structure of a polypeptide that comprises an amino acid sequence of an interacting site with HtrA2 or of a cleavage site by HtrA2 in the amino acid sequence of at least one selected from the group consisting of SHC3, ATF6 and CREBL1 is also suitable as a test compound.

The method of identifying a compound according to the present invention can be specifically carried out by adding a test compound to the in vitro protease assay (Example 2) using, for example, HtrA2 and at least one of SHC3, ATF6 and CREBL1. Mature HtrA2 of an active type can be prepared from E-coli transfected with an appropriate vector containing a mature HtrA2 DNA obtained from the human kidney cDNA library by well known genetic engineering method. CREBL1 can be prepared from a cultured animal cell line (for example, HEK293T cell) transfected with an appropriate vector containing CREBL1 DNA obtained from the human brain cDNA library by well known genetic engineering method. SHC3 can be prepared from a cultured animal cell line (for example, HEK293T cell) transfected with an appropriate vector containing SHC3 DNA obtained from the human brain cDNA library by well known genetic engineering method. ATF6 can be prepared from an animal cell (for example, HEK293T cell) transfected with an appropriate vector containing ATF6 DNA obtained from the human mammary gland cDNA library by well known genetic engineering method. HtrA2, SHC3, ATF6 and CREBL1 are contained in the soluble fractions of lysates of the cells in which these proteins are respectively expressed. SHC3, ATF6 and CREBL1 are preferably expressed as a protein ligated with a tag peptide at the N-terminal or C-terminal, and are favorably purified for use by means of immuno-precipitation using an antibody against the tag peptide. For example, these proteins are subjected to immuno-precipitation using an antibody against the tag peptide, and then captured on a resin (such as protein G sepharose) for use. The in vitro protease assay can be carried out by adding mature HtrA2 to SHC3, CREBL1, or ATF6 captured on a resin, reacting them each other at 37°C overnight, and detecting these proteins by Western blotting. With regard to SHC3 and ATF6, the protease assay may be performed with 4 hours reaction, since SHC3 and ATF6 are degraded by mature HtrA2 in the reaction at 37°C for 4 hours. Detection by Western blotting can be performed using an antibody against a tag peptide ligated to SHC3, CREBL1, or ATF6. Since SHC3, CREBL1, or ATF6 is degraded by HtrA2, the amount of these proteins detected by Western blotting is reduced or disappeared compared with a case without adding HtrA2. In the case that the reduction or disappearance of the amount of SHC3, CREBL1, or ATF6 is inhibited by adding a compound to the reaction of mature HtrA2 with SHC3, CREBL1, or ATF6 in comparison with a case without adding a compound, it can be judged that the test compound inhibits the degradation by HtrA2 of SHC3, CREBL1, or ATF6. The test compound may be previously contacted with SHC3, CREBL1, or ATF6, or mature HtrA2, and then the reaction of the mature HtrA2 with SHC3, CREBL1, or ATF6 may be conducted.

Further, the method of identifying a compound according to the present invention can be specifically carried out by adding a test compound to the intracellular protease assay (Example 3) using, for example, such a cell in which at least one of SHC3, CREBL1, and ATF6 are co-expressed with HtrA2. Co-expression of at least one of SHC3, CREBL1, and ATF6 with HtrA2 can be performed by transfecting a cultured animal cell line (for example, HEK293T cell) with an appropriate vector containing each of SHC3 DNA, CREBL1 DNA, and ATF6 DNA prepared as mentioned above and an appropriate vector containing HtrA2 DNA by a well known genetic engineering method. SHC3, CREBL1, and ATF6 are preferably expressed as the proteins having tag peptides ligated to the N-terminal or C-terminal of these proteins, in order to detect the proteins easily. Active HtrA2 is used for HtrA2. The active HtrA2 suitably used are mature HtrA2, preferably mature HtrA2 (ΔAVPS) that lacks N-temiinal four amino acid residues (AVPS) of mature HtrA2, or mature HtrA2 (GVPS) that has a substitution of alanine among the N-terminal four amino acid residues with glycine. It has been reported that N-terminal four amino acid residues (AVPS) of mature HtrA2, which is a binding motif to IAPs (Inhibitor of apoptosis proteins) family protein acting for inhibiting cell death, bind to IAPs to inhibit the action, thereby to accelerate caspase dependent cell death. Since this action may be likely to give an influence upon the protease assay in a cell, it is preferable to use mature type HrtA2 (ΔAVPS) or mature HtrA2 (GVPS) which does not bind to IAPs. The intracellular protease assay can be performed by culturing a cell in which at least one of SHC3, CREBL1, and ATF6 are co-expressed with HtrA2 at 37°C for 48 hours, together with a test compound, and then lysing the cell to detect SHC3, CREBL1, or ATF6 containing in the cell lysate by Western blotting. Detection by Western blotting can be carried out using an antibody against the tag peptide ligated to SHC3, CREBL1, or ATF6. Since SHC3, CREBL1, or ATF6 is degraded by HtrA2, the amount of these proteins detected by Western blotting is reduced or disappeared compared with a case without adding HtrA2. In the case that the reduction or disappearance of the amount of SHC3, CREBL1, or ATF6 is inhibited by adding a compound in comparison with a case without adding a compound, it can be judged that the test compound inhibits the degradation by HtrA2 of SHC3, CREBL1, or ATF6.

The method of identifying a compound according to the present invention is not limited to the specific examples such as the aforementioned in vitro protease assay or the intracellular protease assay, and an identification method well known for a pharmaceutical screening may be used.

A compound obtained by the method of identifying a compound according to the present invention can be utilized as an agent for inhibiting the degradation by HtrA2 of at least one of SHC3, CREBL1, and ATF6. The compound can be selected in consideration of a balance between the biological usefulness and toxicity to prepare a pharmaceutical composition. In preparation of the pharmaceutical composition, the compound may be used alone or in combination.

The compound that inhibits the degradation by HtrA2 of at least one of SHC3, CREBL1, and ATF6 can be used as an active ingredient for an agent for inhibiting cell death due to the degradation by HtrA2 of at least one of SHC3, CREBL1, and ATF6. Preferably, the compound is useful for an agent for inhibiting cell death due to endoplasmic reticulum stress. More preferably, the compound is useful for an agent for inhibiting neural cell death, still more preferable for an agent for inhibiting neural cell death due to endoplasmic reticulum stress. These inhibitors can be used for conducting a method for inhibiting cell death due to endoplasmic reticulum stress. Preferably, the inhibitors can be used for conducting a method for inhibiting neural cell death due to endoplasmic reticulum stress or brain ischemia.

The compound that inhibits the degradation by HtrA2 of at least one of SHC3, CREBL1, and ATF6 can be used for preparing an agent for preventing, controlling and/or treating diseases attributable to the degradation by HtrA2 of at least one of SHC3, CREBL1, and ATF6. Such a disease can be, for example, a disease accompanied with neural cell death, such as Alzheimer's disease, Parkinson's disease, polyglutamine disease, prion disease, or amyotrophic lateral sclerosis (ALS). These diseases exhibit in most cases a formation of aggregates of abnormal proteins in neural cells, and the relation between neural cell death and endoplasmic reticulum stress in these diseases has been reported (Non-Patent Reference 1). The aforementioned compound may also used for conducting a method for preventing, controlling and/or treating such a disease.

The agent for preventing, treating and/or controlling the diseases according to the present invention can be a pharmaceutical agent prepared by using at least any one of the members consisting of the aforementioned compounds, degradation inhibitor, and cell death inhibitor so as to contain the effective amount thereof. Generally, the pharmaceutical agent is preferably prepared as a pharmaceutical composition that includes one or more kinds of a pharmaceutical carrier.

An amount of the effective ingredient containing in the pharmaceutical preparation can be suitably selected from wide range. In general, a suitable amount may fall within a range of approximately 0.00001 to 70 wt%, preferably approximately 0.0001 to 5 wt%.

As a pharmaceutical carrier, the followings may be used: a filler, extender, binder, wetting agent, disintegrator, lubricant, diluent and excipient that are generally used in accordance with the form of use of the formulation. These can be suitably selected and used in accordance with the administration form of the preparation to be obtained.

More concretely, the pharmaceutical carrier may be water, a pharmaceutically acceptable organic solvent, collagen, polyvinyl alcohol, polyvinylpyrrolidone, carboxyvinyl polymer, sodium alginate, soluble dextran, sodium carboxymethyl starch, pectin, xanthan gum, acacia gum, casein, gelatin, agar, glycerin, propylene glycol, polyethylene glycol, vaseline, paraffin, stearyl alcohol, stearic acid, human serum albumin, mannitol, sorbitol and lactose. One or a combination of two or more kinds of these may be suitably selected and used in accordance with the dosage form of the pharmaceutical composition.

As desired, various components that may be used in conventional protein preparation, such as a stabilizer, bacteriocide, buffer agent, isotonizing agent, chelating agent, surfactant, or pH adjuster, may be suitably contained in the pharmaceutical composition.

As a stabilizer, the followings may be used: human serum albumin, common L-amino acids, sugars and cellulose derivatives. These can be used independently or in combination with a surfactant and the like. Especially, use of these in such a combination may give increased stability of an effective ingredient. An L-amino acid is not particularly limited, and may be any one of glycine, cysteine, glutamic acid and the like. A sugar is not particularly limited, and may be any one of monosaccharides (such as glucose, mannose, galactose and fructose), sugar alcohols (such as mannitol, inositol and xylitol), disaccharides (such as sucrose, maltose and lactose), polysaccharides (dextran, hydroxypropylstarch, chondroitin sulfate and hyaluronic acid), derivatives thereof and so on. A cellulose derivative is not particularly limited, and may be any one of methylcellulose, ethylcellulose, hydroxyethylcellulose, hydroxypropylcellulose, hydroxypropylmethlcellulose, sodium carboxymethylcellulose and the like.

A surfactant is not particularly limited, and can be both an ionic surfactant and a non-ionic surfactant. As a surfactant, the followings may be used: polyoxyethyleneglycol sorbitan alkyl ester system, polyoxyethylene alkyl ether system, sorbitan monoacyl ester system and fatty acid glyceride system.

As a buffer agent, the followings may be used: boric acid, phosphoric acid, acetic acid citric acid, ε-aminocaproic acid, glutamic acid and/or a salt thereof ( for example, an alkaline metal salt and an alkali earth metal salt, such as sodium salt, kalium salt, calcium salt and magnesium salt).

As an isotonizing agent, the followings may be used: sodium chloride, kalium chloride, sugars and glycerin.

As a chelating agent, sodium edentate and citric acid may be used.

The pharmaceutical agent and pharmaceutical composition according to the present invention can be used as a solution preparation. Alternatively, they can be freeze-dried so as to be in good state in preservation and can be used by dissolving them in water, a buffered solution containing saline and the like, and so on and then adjusting to suitable concentration at the time of using.

Suitable dosage ranges of the pharmaceutical composition are not particularly limited, and can be determined according to the following: effectiveness of the ingredients contained therein; the administration form; the route of administration; the type of disease; the characteristics of the subject (e.g., body weight, age, symptomatic conditions and whether being taking other pharmaceutical agent) and the judgment of the physician in charge. In general, a suitable dosage may fall, for example, within a range of about 0.01 *µ*g to 100 mg per 1 kg of the body weight of the subject, and preferably within a range of about 0.1 *µ*g to 1 mg per 1 kg. However, a dosage may be altered using conventional experiments for optimization of a dosage that are well known in the art. The aforementioned dosage can be divided for administration once to several times a day. Alternatively, periodic administration once every few days or few weeks can be employed.

When administering the pharmaceutical composition of the present invention, the pharmaceutical composition may be used alone or may be used together with other compounds or pharmaceutical agents necessary for the treatment.

In terms of a route of administration, it may be either systemic administration or local administration. The route of administration that is appropriate for a particular disease, symptomatic conditions, or other factors should be selected. For example, parenteral administration including normal intravenous injection, intraarterial administration, subcutaneous administration, intracutaneous administration and intramuscular administration can be employed. Oral administration can be also employed. Further, transmucosal administration or dermal administration can be employed.

In terms of an administration form, various forms can be selected in accordance with a treatment purpose. Typically, for example, an administration form of solid formulation may be used, such as a tablet, a pill, powder, powdered drug, fine granule, granule or a capsule, as well as an administration form of liquid formulation such as an aqueous formulation, ethanol formulation, suspension, fat emulsion, liposome formulation, clathrate such as cyclodextrin, syrup and an elixir. These can be further classified according to the administration route into oral formulation, parenteral formulation (drip injection formulation or injection formulation), nasal formulation, inhalant formulation, transvaginal formulation, suppositorial formulation, sublingual agents, eye drop formulation, ear drop formulation, ointment formulation, cream formulation, transdermal absorption formulation, transmucosal absorption formulation and the like, which can be respectively blended, formed and prepared according to conventional methods.

The present invention further provides a reagent kit including at least one member selected from the group consisting of HtrA2, a polynucleotide encoding HtrA2, and a vector containing a polynucleotide encoding HtrA2; and at least one member selected from the group consisting of SHC3, ATF6, CREBL1, a polynucleotide encoding SHC3, ATF6, or CREBL1, and a vector containing the polynucleotide encoding SHC3, ATF6, or CREBL1. The reagent kit can be used, for example, in the identification method of the present invention.

The aforementioned reagent kit may include chemicals that are necessary for carrying out a determination, such as a signal and/or a marker for detecting the degradation of SHC3, ATF6 or CREBL1 by HtrA2, buffer solutions and salts. The reagent kit may also include chemicalss such as stabilizers and/or antiseptic agents. At the time of preparation, methods for preparation may be introduced in accordance with the respective chemicals to be used.

Hereinafter, the present invention may be explained more particularly with examples; however, the present invention is not limited to the following examples.

### Example 1

### (In-silico search for proteins having a function to interact with HtrA2)

The prediction of proteins that have function to interact with HtrA2 was conducted according to the method described in the pamphlet of Intemational Publication No. WO 01/67299. Concretely, the amino acid sequence of HtrA2 was decomposed into oligopeptides having a pre-determined length in order to search in a database for proteins having the amino acid sequence of each of the oligopeptides, or having homologous amino acid sequences to these amino acid sequences. Then, local alignment was conducted between the proteins obtained and HtrA2 to identify proteins having a high local alignment score that might be capable of interacting with HtrA2.

As a result of analysis, CREBL1 and SHClwas identified as a protein being predicted to have a function to interact with HtrA2.

### Example 2

### (In vitro protease assay)

In order to demonstrate experimentally the interaction of HtrA2 with CREBL1, ATF6 that is a family CREBL1, or SHC3 that is a family SHC1, in vitro protease assay was performed.

### <Materials and their preparation>

In this Example, mature HtrA2 (SEQ ID NO: 4) and mature HtrA2 (S306A) (SEQ ID NO: 6) to each of which histidine (His)-tag was ligated at the C-terminal were used as active HtrA2 and inactive HtrA2, respectively. Further, SHC3 (p64) (SEQ ID NO: 16) and SHC3 (p52) to each of which c-Myc-tag was ligated at the C-terminal, and CREBL1 (SEQ ID NO: 18) and ATF6 (SEQ ID NO: 20) to each of which c-Myc-tag was ligated at the N-terminal were used as a protein that was predicted to interact with HtrA2.

### 1. Cloning of mature HtrA2 and mature HtrA2 (S306A), and preparation of expression plasmid

In order to obtain a gene encoding mature HtrA2 (a portion of precursor HtrA2 (DNA nucleotide sequence thereof and amino acid sequence encoded by the DNA are shown in SEQ ID NO: 1 and SEQ ID NO: 2) from 134^{th} to 458^{th} amino acid residues), mature HtrA2 gene was amplified by polymerase chain reaction (PCR) and cloned into pCR-BluntII-TOPO vector (Invitrogen). The PCR was carried out by using Human Kidney QUICK-Clone cDNA (Clontech) as a template , HtrA2-F1 primer (with NdeI site and ATG at the 5' side, SEQ ID NO: 21), HtrA2-RS primer (with XhoI site instead of termination codon, SEQ ID NO: 22), and KOD-plus (Toyobo) as DNA polymerase. The nucleotide sequence was determined by sequencer (Applied Biosystems/Hitachi: ABI3100). The nucleotide sequence of mature HtrA2 DNA obtained in this example, and amino acid sequence encoded by the DNA are shown in SEQ ID NO: 3 and SEQ ID NO: 4, respectively. E-coli expression plasmid for mature HtrA2 was obtained by digesting the cloned mature HtrA2 gene with NdeI and XhoI, and then integrating it into pET24b vector (Novagen).

It has been reported that a mutant (mature HtrA2 (S306A)) with a substitution of 174^{th} serine of mature HtrA2 (which is corresponding to 306^{th} in precursor protein (SEQ ID NO: 2)) with alanine does not exhibit protease activity. Then, E-coli expression plasmid for mature HtrA2 (S306A) that is of inactive type was prepared using mature HtrA2 expression plasmid as a template, with QuickChange XL Site-Directed Mutagenesis kit (Stratagene) using HtrA2-MF primer (SEQ ID NO: 23) and HtrA2-MR primer (SEQ ID NO: 24). The nucleotide sequence was determined by sequencer. The nucleotide sequence of mature HtrA2 (S306A) DNA obtained in this example and amino acid sequence encoded by the DNA are shown in SEQ ID NO: 5 and SEQ ID NO: 6, respectively.

### 2. Expression and purification of mature HtrA2 and mature HtrA2 (S306A)

E-coli expression plasmid for HtrA2 was transfected into into E-coli BL21 Star (DE3) competent cell (Invitrogen) to obtain a transformant The transformant was incubated in LB culture medium (100 ml) at 26°C, and expression induction of mature HtrA2 protein was carried out by adding isopropyl-1-thio-β-D-galactopyranoside (IPTG) to the final concentration of 0.1 mM when absorbance (OD) reached in a range of 0.68 to 0.81. After further incubation for overnight, E-coli that expressed mature HrtA2 was separated by centrifugation and collected. E-coli thus obtained was suspended in lysis buffer A (Lysis buffer: phosphate buffered saline (PBS)/1% TritonX-100, 1*µ*g/ml pepstatin, 5*µ*M E64) followed by treatment with sonicator (15 sec x 10 times) under ice-cooling to disrupt the fungus body. The disrupted fungus body solution was subjected to centrifugation (15,000 rpm, 30 min, 4°C) to separate into a soluble fraction (supematant) and an insoluble fraction. Soluble fraction containing mature HtrA2 was added to ProBondresin (Invitrogen: 1 ml prepacked) that had been pre-equilibrated with 1% Triton X-100 in phosphate buffered saline (PBS), and mixed at 4°C for 30 min, and then washed three times with washing buffer (20 mM sodium phosphate (pH 6.0), 500 mM NaCl). Mature HtrA2 adsorbed by the resin was eluted in stepwise by elution buffer (20 mM, sodium phosphate (pH 6.0) 500 mM NaCl) each containing 50, 100, 200, 350, 500 mM imidazole. Each eluate was subjected to SDS-PASGE to identify the fraction containing mature HtrA2. As a result, mature HtrA2 was eluted by 350 - 500 mM imidazole. The fraction containing mature HtrA2 was dialyzed against 150 mM NaCl, 50 mM Tris-HCl (pH 7.5), followed by centrifugation to remove impurities. The resultant supernatant was concentrated followed by subjecting to the quantitative measurement of protein by Coomassie Plus Protein Assay (PIERCE) while bovine serum albumin (BSA) was used as the standard protein. Similarly, expression and purification of mature HtrA2 (S306A) were performed.

### 3. Cloning of SHC3 (p64), SHC3 (p52), ATF6 and CR E B L 1 , and preparation of expression plasmid

SHC3 (p64) gene was amplified by PCR and cloned into pCR4Blunt-TOPO vector (Invitrogen). The PCR was carried out using Human Brain QUICK-Clone cDNA (Clontech) as a template, SHC3F1 primer (with BamHI site and GCC immediately before ATG, SEQ ID NO: 25), SHC3R1 primer (with XhoI site instead of the termination codon, SEQ ID NO: 26), and KOD-plus as DNA polymerase. The nucleotide sequence was determined by the sequencer. It was found that the nucleotide sequence of SHC3 (p64) DNA thus obtained in this example had a difference in six nucleotides compared with the nucleotide sequence that had already been registered in GenBank with accession number NM_016848. It was revealed that four nucleotides out of the six different nucleotides coincided with the genome sequence: T173A (with amino acid change from Val to Asp); G789A (without amino acid change); A811G (with amino acid change from Thr to Ala); and A1661G (with amino acid change from Gln to Arg). The other two different nucleotides were G291C and A1338G without amino acid change. The nucleotide sequence of SHC3 DNA obtained in this example and the amino acid sequence encoded by the DNA are shown in SEQ ID NO: 15 and SEQ ID NO: 16, respectively. SHC3 animal cell expression plasmid was prepared by digesting the cloned SHC gene with BamHI and XhoI followed by recombination into pCMV-Tag5.

SHC3 (p52), which is a splicing valiant of SHC3, is a protein that is translated from the second ATG (360 bases down stream from the first ATG) within the coding region of SHC3 gene. The expression plasmid of SHC3 (p52) was constructed by digesting the SHC3 (p64) plasmid that had been cloned into pCMV-Tag5A with SacII and XhoI, followed by integrating a SacII XhoI fragment into pCMV-TagSA vector. The integrated SHC3 (p52) gene in the expression plasmid was confirmed by digestion with restriction enzyme.

CREBL1 gene was amplified by PCR and cloned into pCMV-Tag 5. The PCR was carried out using Human Brain cDNA as a template, 83-F primer (with EcoRI site and GCC immediately before ATG, SEQ ID NO: 27), 83-R primer (with XhoI site instead of the termination codon, SEQ ID NO: 28), and KOD-plus as DNA polymerase.

Then, CREBL1 gene was amplified by PCR and cloned into pCR-BluntII-TOPO vector. The PCR was carried out using the CREBL1 gene cloned into pCMV-Tag 5 as a template, ATF6-NF1 primer (with BamHI site instead of ATG, SEQ ID NO: 29), ATF6-NR1 primer (with XhoI site following to termination codon, SEQ ID NO: 30), and KOD-plus as DNA polymerase. The nucleotide sequence was determined by the sequencer. It was found that the nucleotide sequence obtained in this example had a difference in one nucleotide (T450C) compared with the nucleotide sequence of CREBL1 that had already been registered in GenBank with accession number NM_00438. There is no change in the amino acid resulting from this difference. In the meantime, the termination codon was changed from TGA to TAA. It was confirmed that these differences were not due to PCR errors. The nucleotide sequence of CREBL1 DNA obtained in this example and the amino acid sequence encoded by the DNA are shown in SEQ ID NO: 17 and SEQ ID NO: 18, respectively. Animal cell expression plasmid for CREBL1 was prepared by digesting the cloned CREBL1 gene with BamHI and XhoI, and then integrated it into pCMV-Tag 3.

ATF6 gene was amplified by PCR using Mammary Gland QUICK-Clone cDNA (Clontech) as a template, ATF6Fn primer (SEQ ID NO: 31), ATF6Rn primer (SEQ ID NO: 32), and KOD-plus as DNA polymerase. Then, ATF6 gene was further amplified by PCR using the ATF6 gene thus amplified, as a template, using ATF6F1L primer (with EcoRV site immediately before ATG, SEQ ID NO: 33), ATF6R1L primer (with XhoI site immediately after the termination codon, SEQ ID NO: 34), and KOD-plus as DNA polymerase, and then cloned into pCR4Blunt-TOPO vector (Introgen). The nucleotide sequence was determined by the sequencer (SEQ ID NO: 19). It was found that the nucleotide sequence of ATF6 DNA thus obtained in this example had a difference in fifteen nucleotides compared with the nucleotide sequence that had already been registered in GenBank with accession number NM_007348. It was revealed that all of the fifteen different nucleotides coincided with the genome sequence: T105C (without change in amino acid); T199A (accompanied with change in amino acid from Leu to Met); A201 G (accompanied with change in amino acid from Leu to Met); C270T (without change in amino acid); A309G (without change in amino acid); C433G (accompanied with change in amino acid from Pro to Ala); T469C (accompanied with change in amino acid from Ser to Pro); G1228A (accompanied with change in amino acid from Gly to Ser); A1230C (accompanied with change in amino acid from Gly to Ser); C1389T (without change in amino acid); T1416C (without change in amino acid); T1491A without change in amino acid); T1538C (accompanied with change in amino acid from Val to Ala); G1540C (accompanied with change in amino acid from Val to Leu); and 1896A (without change in amino acid). In the meantime, there is a description in SWISS-PROT about changes of all these amino acids in terms of conflict. The nucleotide sequence of ATF6 DNA obtained in this example and the amino acid sequence encoded by the DNA are shown in SEQ ID NO: 19 and SEQ ID NO: 20, respectively.

In order to integrate ATF6 gene into animal cell expression vector, ATF6 gene was amplified by PCR using the aforementioned ATF6 gene having been cloned into pCR4Blunt-TOPO vector as a template, using ATF6F2L primer (with BgIII site immediately before ATG, SEQ ID NO: 35), ATF6R1L primer (SEQ ID NO: 34), and KOD-plus as DNA polymerase, and then cloned into pCR4Blunt-TOPO vector. The nucleotide sequence was determined by sequencer (SEQ ID NO: 19). ATF6 gene thus cloned was digested by BgIII and XhoI to integrate into animal cell expression vector, pCMV-Tag3, digested with BamHI and XhoI.

### 4. Expression of SHC3 (p64), SHC3 (p52), ATF6 and CREBL1

HEK293T cells were cultured to 1.5 x 10⁶ cells /10 cm Dish. Next day, the expression plasmid of SHC3 (p64), SHC3 (p52), CREBL1 or ATF6 (10 µg/Dish) was transfected into HEK293T cells using FuGene 6 (Roche). Forty eight hours later, cells were washed with PBS, followed by adding 1 ml of lysis buffer B (50 mM Tris-HCl (pH 7.6), 150 mM NaCl, 1% Triton X-100, 1% Nonidet P-40 (NP-40), Complete Mini-EDTA (ethylenediamine tetra-acetate) free), and left to stand on ice for 10 min. Then, cells were collected with a scraper, put into a 1.5 ml tube, subjected to sonication under ice-cooling (15 sec x 6 times), left to stand on ice for 20 min, suspended by pipetting, followed by subjecting centrifugation (15,000 rpm, 30 min, 4°C) to separate into a soluble fraction and an insoluble fraction. The soluble fractions were used as a test sample.

### 5. Determination of the activity of mature HtrA2 and mature HtrA2 (S306A)

Determination of the activity of mature HtrA2 and mature HtrA2 (S306A) was carried out with casein zymography and with a protease assay with casein sodium. Casein zymography was carried out according to the protocol (Invitrogen). More concretely, mature HtrA2 and mature HtrA2 (S306A) were mixed under non-reductive condition with equivalent volume of 2 x SDS sample buffer, left to stand at room temperature for 10 min, followed by separation with 4-16% zymogram (Blue casein) gel (Zymogram Gel, Invitrogen). After completion of electrophoresis, they were renatured with 2.5% Triton X-100, and then subjected to a degradation reaction of casein in the developing buffer at 37°C for overnight.

Meanwhile, a protease assay using casein sodium as an substrate was carried out in such that mature HtrA2 or mature HtrA2 (S306A) was mixed with casein sodium in a reaction buffer (150 mM NaCl, 50 mM Tris-HCl (pH 7.5)) so as to the concentration of the former might become 200 *µ*g/ml and the concentration of the latter might become 400 *µ*g/ml., and incubated at 37°C for reactions. A portion of the reaction solution was sampled at three hours and overnight after initiation of the reaction, mixed with equivalent volume of 2 x SDS sample buffer followed by boiling, and then subjected to SDS-PAGE. After that, the degradation of casein sodium was detected by Coomassie Brilliant Blue (CBB) staining.

As a result of the aforementioned investigation, the degradation of casein by mature HtrA2 was observed, but the degradation of casein was not observed with mature HtrA2 (S306A). From these findings, it has been confirmed that mature HtrA2 is of active type having protease activity, but mature HtrA2 (S306A) is of inactive type not exhibiting protease activity.

### <Method>

SHC3 (p64), SHC3 (p52), ATF6 and CREBL1 each of which was captured on the resin by immunoprecipitation was used in the experiments in order to eliminate the influence of proteinaceous impurities contained in the soluble fraction. More specifically, 300 µl of the soluble fraction of SHC3 (p64), SHC3 (p52), ATF6 or CREBL1 was dispensed to six tubes (Nos. 1 to 6), followed by adding 20 µl of protein G sepharose 4 FF (Amersham) of 50% slurry that had been subjected to blocking with BSA, to each of tubes, subjected to overturned mixing at 4°C for 1 hour, and then subjected to centrifizgation (10,000 rpm, 10 sec, 4°C) to collect the supematant, and thus the pre-treatment (preclean) was performed.

The resultant supernatant was mixed with 1.7 µl (2 *µ*g) of anti-Myc antibody (Invitrogen) by overturned mixing at 4°C for 3 hours, followed by adding 20 µl of protein G sepharose 4 FF that had been subjected to blocking with BSA, and then subjected to overturned mixing at 4°C overnight. Thus, SHC3 (p64), SHC3 (p52), ATF6 and CREBL1 each of which was captured on the protein G sepharose 4 FF were prepared. Subsequently, protein G sepharose 4 FF on which SHC3 (p64), SHC3 (p52), ATF6 or CREBL1 was captured was washed with 500 µl of washing buffer (50 mM Tris-HCl (pH 7.5), 150 mM NaCl, 0.01% Triton X-100) five times, and 100 µl of 50 mM Tris-HCl (pH 7.5), 150 mM NaCl, 0.01 % Triton X-100 was added to tubes Nos. 1 and 2, 100 µl of mature HtrA2 solution (50 *µ*g/ml) was added to tubes Nos. 3 and 4, and 100 µl of mature HtrA2 (S306A) solution (50 µg/ml) was added to tubes Nos. 5 and 6, respectively. Tubes Nos. 1, 3 and 5 were subjected to a reaction at 37°C for four hours, tubes Nos. 2, 4, 6 were subjected to a reaction overnight. After the reaction, the centrifugation was carried out to remove the supernatant. The precipitate was subjected to washing/centrifugation for three times with 500 µl of washing buffer, followed by adding 80 µl of 2 x SDS sample buffer (containing 0.1% β-mercaptoethanol) and boiling. Ten µl of the sample thus obtained (80 *µ*l) was subjected to SDS-PAGE and transfered to PVDF membrane onto detect SHC3 (p64), SHC3 (p52), ATF6 and CREBL1 by Western blotting. Anti-Myc antibody (9E10) (Sigma) was used as a primary antibody for detection, and horseradish peroxidase (HRP) labeled anti-mouse IgG antibody (Cell Signaling) was used as a secondary antibody. Detection was carried out using ECL Western Blotting Detection System (Amersham).

### <Results>

It was observed that all of CREBL1, ATF6, and SHC3 (p64) were degraded by mature HtrA2 in vitro. As shown in Fig. 1-A, the band of CREBL1 was significantly reduced after the reaction of active HtrA2 with CREBL1 for overnight (O/N). As shown in Fig. 1-B, the band of ATF6 was significantly reduced after the reaction of active HtrA2 with ATF6 for four hours (4h) or overnight (O/N). As shown in Fig. 1-C, the band of SHC3 (p64) was significantly reduced after the reaction of active HtrA2 with SHC3 (p64) for four hours (4h) or overnight (O/N). Further it was observed that SHC3 (p52) was also degraded by mature HtrA2.

On the other hand, the degradation of CREBL1, ATF6, and SHC3 (p64) by inactive HtrA2 (mature HtrA2 (S306A)) was not observed (Fig. 1-A, Fig. 1-B and Fig. 1-C). Further, the degradation of SHC3 (p52) by inactive HtrA2 (mature HtrA2 (S306A)) was not observed as well.

### Example 3

### (Intracellular protease assay)

Interaction of HtrA2 with CREBL1, ATF6, SHC3 (p64) or SHC3 (p52) was investigated by intracellular protease assay.

### <Materials and their preparations>

It has been reported that N-temlinal four amino acid residues (AVPS) of mature HtrA2, which is a binding motif to IAPs (Inhibitor of apoptosis proteins) family protein acting for inhibiting cell death, bind to IAPs to inhibit the action, thereby to accelerate caspase dependent cell death. Since this action may be likely to give an influence upon the protease assay in a cell. Therefore, in order to inhibit the interaction of mature HtrA2 or mature HtrA2 (S306A) with IAPs, those (ΔAVPS) that lack the N-temlinal four amino acid residues (AVPS) and those (AVPS → GVPS) with a substation of alanine among the four amino acid residues with glycine were prepared for each of mature HtrA2 and mature HtrA2 (S306A). Each of these various types of HtrA2 was prepared as C-terminal FLAG-tagged protein for use.

### 1. Preparation of various types of HtrA2

In order to prepare mature HtrA2 mutants, each gene of mature HtrA2 (ΔAVPS) and mature HtrA2 (GVPS) was amplified by PCR and cloned into pCR-BluntII-TOPO vector. The PCR was carried out using mature HtrA2 as a template, F1 and F2 primers (with SacI site immediately before ATG, SEQ ID NO: 36 and SEQ ID NO: 37, respectively) as the sense primer, HtrA2-RS primer (SEQ ID NO: 22) as the anti-sense primer, and Pfu turbo (STRATAGENE) as the DNA polymerase. The nucleotide sequence was determined by sequencer. Each of animal cell expression plasmids for mature HtrA2 (ΔAVPS) and mature HtrA2 (GVPS) was prepared by digesting each of the cloned mature HtrA2 (ΔAVPS) gene and the cloned mature HtrA2 (GVPS) gene by SacI and XhoI, and then integrated it into pCMV-Tag4. The nucleotide sequence of mature HtrA2 (ΔAVPS) DNA obtained in this example and the amino acid sequence encoded by the DNA are shown in SEQ ID NO: 7 and SEQ ID NO: 8, respectively. Besides, the nucleotide sequence of mature HtrA2 (GVPS) DNA and the amino acid sequence encoded by the DNA are shown in SEQ ID NO: 9 and SEQ ID NO: 10, respectively.

In order to prepare mature HtrA2 (S306A) mutants, with a same manner as mentioned above, each gene of mature HtrA2 (S306A, ΔAVPS) and mature HtrA2 (S306A, GVPS) was amplified by PCR using mature HtrA2 (S306A) as a template, and then cloned into pCR-BluntII-TOPO vector. Each of animal cell expression plasmids of mature HtrA2 (S306A, ΔAVPS) and mature HtrA2 (S306A, GVPS) was prepared by digesting each of the cloned mature HtrA2 (S306A, ΔAVPS) and the cloned mature HtrA2 (S306A, GVPS) by SacI and XhoI, and then integrated it into pCMV-Tag4. The nucleotide sequence of mature HtrA2 (S306A, ΔAVPS) DNA obtained in this example and the amino acid sequence encoded by the DNA are shown in SEQ ID NO: 11 and SEQ ID NO: 12, respectively. Besides, the nucleotide sequence of mature HtrA2 (S306A, GVPS) DNA and the amino acid sequence encoded by the DNA are shown in SEQ ID NO: 13 and SEQ ID NO: 14, respectively.

### 2. Determination of enzyme activity of various types of HtrA2

The enzyme activity of the various types of HtrA2 each of which was expressed in a cultured cell was determined by the protease assay using casein sodium as a substrate (see Example 2). As a result, it has been revealed that both mature HtrA2 (ΔAVPS) and mature HtrA2 (GVPS) are of active type having protease activity and that both mature HtrA2 (S306A, ΔAVPS) and mature HtrA2 (S306A, GVPS) are of inactive type without exhibiting protease activity.

### 3. Preparation of animal cell expression vectors for SHC3 (p64), SHC3 (p52), ATF6 and CREBL1

The vectors that were prepared by the same procedures used in Example 2 were used.

### <Method>

HEK293T cells were cultured to 1.5 x 10⁵ cells /6 cm Dish. Next day, the expression plasmid of each protein in interest (SHC3 (p64), SHC (p52), ATF6, and CREBL1) was transfected into HEK293T cells together with the expression plasmid of various types of HtrA2 using FuGene 6 (Roche). Each of the expression plasmid of various HtrA2 at 1 *µ*g/Dish was added in combination with any one of the expression plasmid of the protein in interest at 1 µg/Dish. Forty eight hours later, the cells in which the various types of HtrA2 were co-expressed with SHC3 (p64) or SHC (p52) were added with 400 µl of lysis buffer B, and subjected to centrifugation to collect supernatant that was then added with an equivalent volume of 2 x SDS sample buffer (containing 0.1 % β-mercaptoethanol) to use as a test sample. In the meanwhile, the cells in which the various types of HtrA2 were co-expressed with CREBL1 or ATF6 were added with 200 µl of lysis buffer B and an equivalent volume of 2 x SDS sample buffer (containing 0.1% β-mercaptoethanol), and then subjected to sonication followed by boiling to use as a test sample

Expression of SHC3 (p64), SHC (p52), ATF6, and CREBL1 and the degradation of these proteins were detected by Western blotting using 10 *µ*l of the test sample thus obtained. Anti-c-Myc (9E10) antibody was used as a primary antibody for detection, and horseradish peroxidase (HRP) conjugated anti-mouse IgG antibody (Cell Signaling) was used as a secondary antibody. Detection was carried out using ECL Western Blotting Detection System.

### <Results>

In the analysis using cells in which the active mutant (mature HtrA2 (ΔAVPS) or mature HtrA2 (GVPS)) was co-expressed with CREBL1, the band of CREBL1 was significantly reduced (upper panel of Fig 2-A). In the analysis using cells in which the active mutant was co-expressed with ATF6, the band of ATF6 was significantly reduced (upper panel of Fig 2-B). In the analysis using cells in which the active mutant was co-expressed with SHC3 (p64), the band of SHC3 (p64) was significantly reduced (upper panel of Fig 2-C). In the analysis using cells in which the active mutant was co-expressed with SHC3 (p52), the band of SHC3 (p52) was significantly reduced (upper panel of Fig 2-D). On the other hand, in the analysis using cells in which the inactive mutant (mature HtrA2 S306 (ΔAVPS) or mature HtrA2 S306 (GVPS)) was co-expressed with CREBL1, the reduction of the band of CREBL1 was not observed (upper panel of Fig. 2-A). In the analysis using cells in which the inactive mutant was co-expressed with ATF6, the reduction of the band of ATF6 was not observed (upper panel of Fig. 2-B). In the analysis using cells in which the inactive mutant was co-expressed with SHC3 (p64), the reduction of the band of SHC3 (p64) was not observed (upper panel of Fig. 2-C). In the analysis using cells in which the inactive mutant was co-expressed with SHC3 (p52), the reduction of the band of SHC3 (p52) was not observed (upper panel of Fig. 2-D). The expression of each HtrA2 mutant in the cells was almost same among the cells (lower panel of Fig. 2-A, 2-B, 2-C and 2-D).

As mentioned above, it has been revealed that all of CREBL1, ATF6, SHC3 (p64), and SHC3 (p52) are degraded in the cells by active mature HtrA2 (ΔAVPS) or by active mature HtrA2 (GVPS). On the other hand, any of CREBL1, ATF6, SHC3 (p64), and SHC3 (p52) is not degraded by inactive mature HtrA2 (S306A, ΔAVPS) or by inactive mature HtrA2 (S306A, GVPS).

### Example 4

### (Investigation of degradation pattern by HtrA2)

Degradation pattern of CREBL1, SHC3 (p64), or SHC3 (p52) by HtrA2 was investigated. The investigation was performed by in vitro protease assay with HtrA2 using CREBL1, SHC3 (p64), or SHC3 (p52) which is labeled with biotin.

### <Materials and their preparations>

### 1. Mature HtrA2 and mature HtrA2 (S306A)

Mature HtrA2 and mature HtrA2 (S306A) were prepared by similar procedures as used in Example 2 for use.

### 2. animal cell expression plasmid for CREBL1, SHC3 (p64) and SHC3 (p52)

The animal cell expression plasmid for CREBL1 was prepared by digesting CREBL1, which had been cloned into pCR-Bluntfl-TOPO vector by similar procedures as used in Example 2, with BamHI and XhoI, and then integrated it into pcDNA3.1/His.
The animal cell expression plasmid for SHC3 (p64) was prepared by digesting SHC3 (p64), which had been cloned into pCR4Blunt-TOPO vector by similar procedures as used in Example 2, with EcoRI and XhoL and then integrated it into pcDNA3.1/V5-His.
The animal cell expression plasmid for SHC3 (p52) was prepared by digesting SHC3 (p52), which had been cloned into pCMV-Tag5 by similar procedures as used in Example 2, with PvuII and XhoI, and then ligated it to pcDNA3.1/V5-His digested with EcoRV and XhoI.

### 3. Preparation of biotinylated CREBL1, biotinylated SHC3 (p64), and biotinylated SHC3 (p52) using in vitro translation reaction system

The preparation of biotinylated CREBL1, biotinylated SHC3 (p64), and biotinylated SHC3 (p52) was performed by in vitro translation reaction system (T_{N}T® Transcription/Translation System; Promega) using rabbit reticulocyte lysate.
More concretely, first, 1.5 µl of the expression plasmid, 1 µl of 1 mM methionine, 1 µl of biotinylated lysine tRNA (Promega), and 6.5 µl of nuclease free water were added to 40 µl of in vitro translation reaction solution (T_{N}T® Quick Master Mix) to obtain total volume of 50 *µ*l, and reacted for at 30°C for 1.5 hours. Following this, 12.5 *µ*l of 50 *µ*l reaction solution was sampled, followed by adding with 2 x SDS sample buffer and boiling, to detect the expression of biotinylated CREBL1, biotinylated SHC3 (p64), and biotinylated SHC3 (p52) by Western blotting. The detection was carried out using streptavidin HRP (Promega) and Transcend^{™} Chemiluminescent substrate (Transcend^{™} Non-Radioactive Translation detection system; Promega). As a result, biotinylation of these proteins was confirmed.

### <Methods>

The reaction solution was used as the sample for protease assay. 12.5 µl each of the reaction solution was dispensed to three tubes (Nos. 2 to 4). 25 µl of 50 mM Tris-HCl (pH 7.5), 150 mM NaCl, 0.01% Triton X-100 was added as the control to tube No.2, 25 µl of 200 µg/ml mature HtrA2 solution was added to tube No.3, and 25 µl of 200*µ*g/ml mature HtrA2 (S306A) solution was added to tube No.4, respectively, and mixed. The solution in each tube (Nos. 2 to 4) was further divided into two tubes so as to the tubes include an equivalent volume of solution, while one tube was subjected to reaction at 37°C for four hours and the other tube was subjected to reaction at 37°C overnight. After the reaction, 2 x SDS sample buffer was added to each of tubes, and subsequently subjected to boiling, to detect the degradation pattern of biotinylated CREBL1, biotinylated SHC3 (p64), and biotinylated SHC3 (p52) by Western blotting.

The detection of the degradation was carried out using streptavidin HRP and Transcend^{™} Chemiluminescent substrate while biotinylated lysine residue in each protein was used as the index.

Further, in order to determine the degradation site, the membrane used for Western blotting was subjected to treatment for removing the streptavidin HRP, and then used for detecting the degradation pattern of biotinylated CREBL1, biotinylated SHC3 (p64), and biotinylated SHC3 (p52) using an antibody against the tag ligated to each protein. Anti-V5 antibody (Invitrogen) and HRP labeled anti-mouse IgG antibody (Cell Signaling) were used as a primary antibody and a secondary antibody, respectively, for detecting SHC3. Anti-Xpress antibody (Invitrogen) and HRP labeled anti-mouse IgG antibody (Cell Signaling) were used as a primary antibody and a secondary antibody, respectively, for detecting CREBL1. The detection of degradation was carried out using ECL Western Blotting Detection Reagents (Amersham).

### <Results>

Results of the study for the degradation pattern of CREBL1, SHC3 (p64), or SHC3 (p52) by mature HtrA2 using biotinylated lysine residue in the protein as an index are shown in Fig. 3-A, Fig. 3-B and Fig. 3-C, respectively. Similarly, results of the study for the degradation pattern of CREBL1, SHC3 (p64), or SHC3 (p52) using a tag ligated to the N-terminal or C-terminal of the protein as an index are shown in Fig. 4-A, Fig. 4-B and Fig. 4-C, respectively.

In the study for the degradation pattern using biotinylated lysine residue as an index, remarkable reduction of the band of biotinylated CREBL1 was observed, and further, the band that was supposed to be a degradation product of CREBL1 was detected around 50 kDa (Fig. 3-A). However, in the study for the degradation pattern using the anti-tag antibody, the band that was supposed to be a degradation product of CREBL1 which was detected around 50 kDa in Fig. 3-A could not be detected. Moreover, any band indicating the degradation product of CREBL1 with different size was not detected (Fig. 4-A). From this finding, it is believed that CREBL1 was degraded by HtrA2 at several sites.

In the study for the degradation pattern using biotinylated lysine residue as an index, remarkable reduction of the band of biotinylated SHC3 (p64) was observed, and further, the bands that were supposed to be a degradation products of SHC3 (p64) were detected around 70 kDa, 40 kDa, and 30 kDa (Fig. 3-B). In addition, the same bands were detected in the study for the degradation pattern using the anti-tag antibody (Fig. 4-B). From this finding, it can be considered that SHC3 (p64) may be degraded by mature HtrA2 at several sites.

In the study for the degradation pattern using biotinylated lysine residue as an index, remarkable reduction of the band of biotinylated SHC3 (p52) was observed, but the band that was supposed to be a degradation product of SHC3 (p52) was not detected (Fig. 3-C). However, in the study for the degradation pattern using the anti-tag antibody, the band that was supposed to be a degradation products of SHC3 (p52) was detected around 40kDa (Fig. 4-C). From this finding, it is believed that SHC3 (p52) was degraded by HtrA2 at several sites.

As described above, the degradation of the aforementioned protein by HtrA2 was detected by detecting a labeled chemical that was used for labeling the inside of the protein. From this finding, it has been revealed that the degradation of the protein by HtrA2 identified by detection of the tag ligated to the N-terminal of each protein (Examples 2 and 3) is not an apparent degradation due to the cleavage of the tag, but is a degradation resulting from the action of HtrA2 on the inside of each protein.

### INDUSTRIAL APPLICABILITY

The present invention can be utilized in inhibition of cell death, such as neural cell death, attributable to the degradation by HtrA2 of at least one of SHC3, ATF6 and CREBL1, and in prevention, treatment, or control of the diseases accompanied with neural cell death, such as neurodegenerative diseases, attributable to the same. Thus, the present invention is extremely useful in the pharmaceutical fields. Further, the present invention can be utilized in the research field, such as the investigation of cell death caused by HtrA2, for example, cell death due to the endoplasmic reticulum stress, or neural cell death.

### SEQUENCE LISTING FREE TEXT

SEQ ID NO: 1: DNA encoding HtrA2 precursor protein
SEQ ID NO: 2: HtrA2 precursor protein
SEQ ID NO: 3: DNA encoding mature HtrA2
SEQ ID NO: 4: mature HtrA2
SEQ ID NO: 5: Polynucleotide consisting of the same nucleotide sequence of SEQ ID NO: 3, wherein the nucleotide of position 520 is g; DNA encoding mature HtrA2 (S306A)
SEQ ID NO: 6: Polypeptide consisting of the same amino acid sequence of SEQ ID NO: 4, wherein the 174th amino acid residue is substituted by Ala; mature HtrA2 (S306A)
SEQ ID NO: 7: Polynucleotide consisting of the same nucleotide sequence of SEQ ID NO: 3, wherein the nucleotides of position 4-15 are deleted; DNA encoding mature HtrA2 (delta AVPS)
SEQ ID NO: 8: Polypeptide consisting of the same amino acid sequence of SEQ ID NO: 4, wherein the amino acid residues from the 2nd to the 5th are deleted; mature HtrA2 (delta AVPS)
SEQ ID NO: 9: Polynucleotide consisting of the same nucleotide sequence of SEQ ID NO: 3, wherein the nucleotide of position 5 is g; DNA encoding mature HtrA2 (GVPS)
SEQ ID NO: 10: Polypeptide consisting of the same amino acid sequence of SEQ ID NO: 4, wherein the 2nd amino acid residue is substituted by Gly; mature HtrA2 (GVPS)
SEQ ID NO: 11: Polynucleotide consisting of the same nucleotide sequence of SEQ ID NO: 5, wherein the nucleotides of position 4-15 are deleted; DNA encoding mature HtrA2 (S306A, delta AVPS)
SEQ ID NO: 12: Polypeptide consisting of the same amino acid sequence of SEQ ID NO: 6, wherein the amino acid residues from the 2nd to the 5th are deleted; mature HtrA2 (S306A, delta AVPS)
SEQ ID NO: 13: Polynucleotide consisting of the same nucleotide sequence of SEQ ID NO: 5, wherein the nucleotide of position 5 is g; DNA encoding mature HtrA2 (S306A, GVPS)
SEQ ID NO: 14: Polypeptide consisting of the same amino acid sequence of SEQ ID NO: 6, wherein the 2nd amino acid residue is substituted by Gly; mature HtrA2 (S306A, GVPS)
SEQ ID NO: 15: DNA encoding SHC3
SEQ ID NO:16:SHC3
SEQ ID NO: 17: DNA encoding CREBL1
SEQ ID NO: 18: CREBL1
SEQ ID NO: 19: DNA encoding ATF6
SEQ ID NO: 20: ATF6
SEQ ID NO: 21: Designed polynucleotide based on the nucleotide sequence of SEQ ID NO: 3 for use as a primer to obtain mature HtrA2 DNA
SEQ ID NO: 22: Designed polynucleotide based on the nucleotide sequence of SEQ ID NO: 3 for use as a primer to obtain mature HtrA2 DNA
SEQ ID NO: 23: Designed polynucleotide based on the nucleotide sequence of SEQ ID NO: 3 for use as a primer to obtain mature HtrA2 (S306A) DNA
SEQ ID NO: 24: Designed polynucleotide based on the nucleotide sequence of SEQ ID NO: 3 for use as a primer to obtain mature HtrA2 (S306A) DNA
SEQ ID NO: 25: Designed polynucleotide based on the nucleotide sequence of SEQ ID NO: 15 for use as a primer to obtain SHC3 DNA
SEQ ID NO: 26: Designed polynucleotide based on the nucleotide sequence of SEQ ID NO: 15 for use as a primer to obtain SHC3 DNA
SEQ ID NO: 27: Designed polynucleotide based on the nucleotide sequence of SEQ ID NO: 17 for use as a primer to obtain CREBL1 DNA
SEQ ID NO: 28: Designed polynucleotide based on the nucleotide sequence of SEQ ID NO: 17 for use as a primer to obtain CREBL1 DNA
SEQ ID NO: 29: Designed polynucleotide based on the nucleotide sequence of SEQ ID NO: 17 for use as a primer to obtain CREBL1 DNA
SEQ ID NO: 30: Designed polynucleotide based on the nucleotide sequence of SEQ ID NO: 17 for use as a primer to obtain CREBL1 DNA
SEQ ID NO: 31: Designed polynucleotide based on the nucleotide sequence of SEQ ID NO: 19 for use as a primer to obtain ATF6 DNA
SEQ ID NO: 32: Designed polynucleotide based on the nucleotide sequence of SEQ ID NO: 19 for use as a primer to obtain ATF6 DNA
SEQ ID NO: 33: Designed polynucleotide based on the nucleotide sequence of SEQ ID NO: 19 for use as a primer to obtain ATF6 DNA
SEQ ID NO: 34: Designed polynucleotide based on the nucleotide sequence of SEQ ID NO: 19 for use as a primer to obtain ATF6 DNA
SEQ ID NO: 35: Designed polynucleotide based on the nucleotide sequence of SEQ ID NO: 19 for use as a primer to obtain ATF6 DNA
SEQ ID NO: 36: Designed polynucleotide based on the nucleotide sequence of SEQ ID NO: 3 for use as a primer to obtain mature HtrA2 (delta AVPS) DNA
SEQ ID NO: 37: Designed polynucleotide based on the nucleotide sequence of SEQ ID NO: 3 for use as a primer to obtain mature HtrA2 (GVPS) DNA

## Claims

1. A method for inhibiting neural cell death, comprising inhibiting the degradation by HtrA2 (high temperature requirement protein A2) of at least one of SHC3 (src homology 2 domain containing transforming protein C3), ATF6 (activating transcription factor 6), and CREBL1 (cAMP responsive element binding protein-like 1).

2. A method for inhibiting neural cell death, comprising using one or more compounds that inhibit the degradation by HtrA2 of at least one of SHC3, ATF6 and CREBL1.

3. The method for inhibiting neural cell death according to claim 1 or 2, in which the neural cell death is neural cell death attributable to brain ischemia.

4. A method for preventing, treating or controlling brain ischemia or neurodegenerative disease, comprising inhibiting the degradation by HtrA2 of at least one of SHC3, ATF6 and CREBL1.

5. The preventing, treating, or controlling method according to claim 4, in which the neurodegenerative disease is Alzheimer's disease, Parkinson's disease, polyglutamine disease, prion disease, or amyotrophic lateral sclerosis.

6. A method of identifying a compound that inhibits the degradation by HtrA2 of at least one of SHC3, ATF6 and CREBL1, comprising contacting HtrA2 and at least one of SHC3, ATF6 and CREBL1 with a compound under conditions that allow the degradation by HtrA2 of at least one of SHC3, ATF6 and CREBL1; introducing a system using a signal and a marker capable of detecting at least one of SHC3, ATF6 and CREBL1; detecting the presence or absence and/or change of the signal and the marker; and determining whether the compound inhibits the degradation of at least one of SHC3, ATF6 and CREBL1.

7. A method of identifying a compound that inhibits the degradation by HtrA2 of at least one of SHC3, ATF6 and CREBL1, comprising contacting HtrA2 and at least one of SHC3, ATF6 and CREBL1 with a compound under conditions that allow the degradation by HtrA2 of at least one of SHC3, ATF6 and CREBL1; detecting the presence or absence of at least one of SHC3, ATF6 and CREBL1, and/or measuring the change of the amount thereof; or detecting the presence or absence of the degradation product of at least one of SHC3, ATF6 and CREBL1, and/or measuring the change of the amount thereof ; and determining whether the compound inhibits the degradation of at least one of SHC3, ATF6 and CREBL1.

8. An agent for inhibiting neural cell death, comprising one or more compounds that inhibit the degradation by HtrA2 of at least one of SHC3, ATF6 and CREBL1.

9. The agent for inhibiting neural cell death according to claim 8, wherein the neural cell death is neural cell death attributable to brain ischemia.

10. An agent for preventing, treating or controlling brain ischemia or neurodegenerative disease, comprising one or more compounds that inhibit the degradation by HtrA2 of at least one of SHC3, ATF6 and CREBL1.

11. The agent for preventing, treating or controlling neurodegenerative disease according to claim 10, wherein the neurodegenerative disease is Alzheimer's disease, Parkinson's disease, polyglutamine disease, prion disease, or amyotrophic lateral sclerosis.

12. A reagent kit, comprising at least one selected from the group consisting of HtrA2, a polynucleotide encoding HtrA2, and a vector containing the polynucleotide encoding HtrA2; and at least one selected from the group consisting of SHC3, ATF6, CREBL1, a polynucleotide encoding at least one of SHC3, ATF6 and CREBL1, and a vector containing the polynucleotide encoding at least one of SHC3, ATF6 and CREBL1.


SEQUENCE LISTING

<110> DAIICHI PHARMACEUTICAL CO., LTD.

<120> Inhibition of nerve cell death by inhibiting degradation of SHC3, ATF6 or CREBL1 by HtrA2 and method of ameliorating neurodegenerative diseases

<130> DAII-002-EP

<140> 04788406.9
<141> 2004-09-30

<150> JP P2003-342588
<151> 2003-09-30

<160> 37

<170> PatentIn version 3.1

<210> 1
<211> 1377
<212> DNA
<213> Homo sapiens

<220>
<221> misc_feature
<223> DNA that codes for HtrA2 precursor protein

<400> 1
atggctgcgc cgagggcggg gcggggtgca ggctggagcc ttcgggcatg gcgggctttg 60

gggggcattc gctgggggag gagaccccgt ttgacccctg acctccgggc cctgctgacg 120

tcaggaactt ctgacccccg ggcccgagtg acttatggga cccccagtct ctgggcccgg 180

ttgtctgttg gggtcactga accccgagca tgcctgacgt ctgggacccc gggtccccgg 240

gcacaactga ctgcggtgac cccagatacc aggacccggg aggcctcaga gaactctgga 300

acccgttcgc gcgcgtggct ggcggtggcg ctgggcgctg ggggggcagt gctgttgttg 360

ttgtggggcg gggggtcggg gtcctccggcc gtcctcgccg ccgtccctag cccgccgccc 420

gcttctcccc ggagtcagta caacttcatc gcagatgtgg tggagaagac agcacctgcc 480

gtggtctata tcgagatcct ggaccggcac cctttcttgg gccgcgaggt ccctatctcg 540

aacggctcag gattcgtggt ggctgccgat gggctcattg tcaccaacgc ccatgtggtg 600

gctgatcggc gcagagtccg tgtgagactg ctaagcggcg acacgtatga ggccgtggtc 660

acagctgtgg atcccgtggc agacatcgca acgctgagga ttcagactaa ggagcctctc 720

cccacgctgc ctctgggacg ctcagctgat gtccggcaag gggagtttgt tgttgccatg 780

ggaagtccct ttgcactgca gaacacgatc acatccggca ttgttagctc tgctcagcgt 840

ccagccagag acctgggact cccccaaacc aatgtggaat acattcaaac tgatgcagct 900

attgattttg gaaactctgg aggtcccctg gttaacctgg atggggaggt gattggagtg 960

aacaccatga aggtcacagc tggaatctcc tttgccatcc cttctgatcg tcttcgagag 1020

tttctgcatc gtggggaaaa gaagaattcc tcctccggaa tcagtgggtc ccagcggcgc 1080

tacattgggg tgatgatgct gaccctgagt cccagcatcc ttgctgaact acagcttcga 1140

gaaccaagct ttcccgatgt tcagcatggt gtactcatcc ataaagtcat cctgggctcc 1200

cctgcacacc gggctggtct gcggcctggt gatgtgattt tggccattgg ggagcagatg 1260

gtacaaaatg ctgaagatgt ttatgaagct gttcgaaccc aatcccagtt ggcagtgcag 1320

atccggcggg gacgagaaac actgacctta tatgtgaccc ctgaggtcac agaatga 1377

<210>  2
<211>  458
<212>  PRT
<213>  Homo sapiens

<220>
<221>  misc_feature
<223>  HtrA2 precursor protein


<400>  2

<210> 3
<211> 981
<212> DNA
<213> Homo sapiens

<220>
<221> misc_feature
<223> DNA that codes for mature HtrA2

<400> 3
atggccgtcc ctagcccgcc gcccgcttct ccccggagtc agtacaactt catcgcagat 60

gtggtggaga agacagcacc tgccgtggtc tatatcgaga tcctggaccg gcaccctttc 120

ttgggccgcg aggtccctat ctcgaacggc tcaggattcg tggtggctgc cgatgggctc 180

attgtcacca acgcccatgt ggtggctgat cggcgcagag tccgtgtgag actgctaagc 240

ggcgacacgt atgaggccgt ggtcacagct gtggatcccg tggcagacat cgcaacgctg 300

aggattcaga ctaaggagcc tctccccacg ctgcctctgg gacgctcagc tgatgtccgg 360

caaggggagt ttgttgttgc catgggaagt ccctttgcac tgcagaacac gatcacatcc 420

ggcattgtta gctctgctca gcgtccagcc agagacctgg gactccccca aaccaatgtg 480

gaatacattc aaactgatgc agctattgat tttggaaact ctggaggtcc cctggttaac 540

ctggatgggg aggtgattgg agtgaacacc atgaaggtca cagctggaat ctcctttgcc 600

atcccttctg atcgtcttcg agagtttctg catcgtgggg aaaagaagaa ttcctcctcc 660

ggaatcagtg ggtcccagcg gcgctacatt ggggtgatga tgctgaccct gagtcccagc 720

atccttgctg aactacagct tcgagaacca agctttcccg atgttcagca tggtgtactc 780

atccataaag tcatcctggg ctcccctgca caccgggctg gtctgcggcc tggtgatgtg 840

attttggcca ttggggagca gatggtacaa aatgctgaag atgtttatga agctgttcga 900

acccaatccc agttggcagt gcagatccgg cggggacgag aaacactgac cttatatgtg 960

acccctgagg tcacagaatg a 981

<210> 4
<211> 326
<212> PRT
<213> Homo sapiens

<220>
<221> misc_feature
<223> mature HtrA2

<400> 4

Met Ala Val Pro Ser Pro Pro Pro Ala Ser Pro Arg Ser Gln Tyr Asn
1               5                   10                  15

Phe Ile Ala Asp Val Val Glu Lys Thr Ala Pro Ala Val Val Tyr Ile
            20                  25                  30

Glu Ile Leu Asp Arg His Pro Phe Leu Gly Arg Glu Val Pro Ile Ser
        35                  40                  45

Asn Gly Ser Gly Phe Val Val Ala Ala Asp Gly Leu Ile Val Thr Asn
    50                  55                  60

Ala His Val Val Ala Asp Arg Arg Arg Val Arg Val Arg Leu Leu Ser
65                  70                  75                  80

Gly Asp Thr Tyr Glu Ala Val Val Thr Ala Val Asp Pro Val Ala Asp
                85                  90                  95

Ile Ala Thr Leu Arg Ile Gln Thr Lys Glu Pro Leu Pro Thr Leu Pro
            100                 105                 110

Leu Gly Arg Ser Ala Asp Val Arg Gln Gly Glu Phe Val Val Ala Met
        115                 120                 125

Gly Ser Pro Phe Ala Leu Gln Asn Thr Ile Thr Ser Gly Ile Val Ser
    130                 135                 140

Ser Ala Gln Arg Pro Ala Arg Asp Leu Gly Leu Pro Gln Thr Asn Val
145                 150                 155                 160

Glu Tyr Ile Gln Thr Asp Ala Ala Ile Asp Phe Gly Asn Ser Gly Gly
                165                 170                 175

Pro Leu Val Asn Leu Asp Gly Glu Val Ile Gly Val Asn Thr Met Lys
            180                 185                 190

Val Thr Ala Gly Ile Ser Phe Ala Ile Pro Ser Asp Arg Leu Arg Glu
        195                 200                 205

Phe Leu His Arg Gly Glu Lys Lys Asn Ser Ser Ser Gly Ile Ser Gly
    210                 215                 220

Ser Gln Arg Arg Tyr Ile Gly Val Met Met Leu Thr Leu Ser Pro Ser
225                 230                 235                 240

Ile Leu Ala Glu Leu Gln Leu Arg Glu Pro Ser Phe Pro Asp Val Gln
                245                 250                 255

His Gly Val Leu Ile His Lys Val Ile Leu Gly Ser Pro Ala His Arg
            260                 265                 270

Ala Gly Leu Arg Pro Gly Asp Val Ile Leu Ala Ile Gly Glu Gln Met
        275                 280                 285

Val Gln Asn Ala Glu Asp Val Tyr Glu Ala Val Arg Thr Gln Ser Gln
    290                 295                 300

Leu Ala Val Gln Ile Arg Arg Gly Arg Glu Thr Leu Thr Leu Tyr Val
305                 310                 315                 320

Thr Pro Glu Val Thr Glu
                325

<210>  5
<211>  981
<212>  DNA
<213>  Artificial

<220>
<223>  Polynucleotide consisting of the same base sequence of SEQ ID NO:
       3 wherein the nucleotide of position 520 is g

<220>
<221>  misc_feature
<223>  DNA that codes for mature HtrA2(S306A)

<400>  5
atggccgtcc ctagcccgcc gcccgcttct ccccggagtc agtacaactt catcgcagat      60

gtggtggaga agacagcacc tgccgtggtc tatatcgaga tcctggaccg gcaccctttc     120

ttgggccgcg aggtccctat ctcgaacggc tcaggattcg tggtggctgc cgatgggctc     180

attgtcacca acgcccatgt ggtggctgat cggcgcagag tccgtgtgag actgctaagc     240

ggcgacacgt atgaggccgt ggtcacagct gtggatcccg tggcagacat cgcaacgctg     300

aggattcaga ctaaggagcc tctccccacg ctgcctctgg gacgctcagc tgatgtccgg     360

caaggggagt ttgttgttgc catgggaagt ccctttgcac tgcagaacac gatcacatcc     420

ggcattgtta gctctgctca gcgtccagcc agagacctgg gactccccca aaccaatgtg     480

gaatacattc aaactgatgc agctattgat tttggaaacg ctggaggtcc cctggttaac     540

ctggatgggg aggtgattgg agtgaacacc atgaaggtca cagctggaat ctcctttgcc 600

atcccttctg atcgtcttcg agagtttctg catcgtgggg aaaagaagaa ttcctcctcc 660

ggaatcagtg ggtcccagcg gcgctacatt ggggtgatga tgctgaccct gagtcccagc 720

atccttgctg aactacagct tcgagaacca agctttcccg atgttcagca tggtgtactc 780

atccataaag tcatcctggg ctcccctgca caccgggctg gtctgcggcc tggtgatgtg 840

attttggcca ttggggagca gatggtacaa aatgctgaag atgtttatga agctgttcga 900

acccaatccc agttggcagt gcagatccgg cggggacgag aaacactgac cttatatgtg 960

acccctgagg tcacagaatg a 981

<210> 6
<211> 326
<212> PRT
<213> Artificial

<220>
<223> Polypeptide consisting of the same amino acid sequence of SEQ ID
 NO:4 wherein the 174th amino acid residue is substituted by Ala

<220>
<221> misc_feature
<223> mature HtrA2(S306A)

<400> 6

Met Ala Val Pro Ser Pro Pro Pro Ala Ser Pro Arg Ser Gln Tyr Asn
1               5                   10                  15

Phe Ile Ala Asp Val Val Glu Lys Thr Ala Pro Ala Val Val Tyr Ile
            20                  25                  30

Glu Ile Leu Asp Arg His Pro Phe Leu Gly Arg Glu Val Pro Ile Ser
        35                  40                  45

Asn Gly Ser Gly Phe Val Val Ala Ala Asp Gly Leu Ile Val Thr Asn
    50                  55                  60

Ala His Val Val Ala Asp Arg Arg Arg Val Arg Val Arg Leu Leu Ser
65                  70                  75                  80

Gly Asp Thr Tyr Glu Ala Val Val Thr Ala Val Asp Pro Val Ala Asp
                85                  90                  95

Ile Ala Thr Leu Arg Ile Gln Thr Lys Glu Pro Leu Pro Thr Leu Pro
            100                 105                 110

Leu Gly Arg Ser Ala Asp Val Arg Gln Gly Glu Phe Val Val Ala Met
        115                 120                 125

Gly Ser Pro Phe Ala Leu Gln Asn Thr Ile Thr Ser Gly Ile Val Ser
    130                 135                 140

Ser Ala Gln Arg Pro Ala Arg Asp Leu Gly Leu Pro Gln Thr Asn Val
145                 150                 155                 160

Glu Tyr Ile Gln Thr Asp Ala Ala Ile Asp Phe Gly Asn Ala Gly Gly
                165                 170                 175

Pro Leu Val Asn Leu Asp Gly Glu Val Ile Gly Val Asn Thr Met Lys
            180                 185                 190

Val Thr Ala Gly Ile Ser Phe Ala Ile Pro Ser Asp Arg Leu Arg Glu
        195                 200                 205

Phe Leu His Arg Gly Glu Lys Lys Asn Ser Ser Ser Gly Ile Ser Gly
    210                 215                 220

Ser Gln Arg Arg Tyr Ile Gly Val Met Met Leu Thr Leu Ser Pro Ser
225                 230                 235                 240

Ile Leu Ala Glu Leu Gln Leu Arg Glu Pro Ser Phe Pro Asp Val Gln
                245                 250                 255

His Gly Val Leu Ile His Lys Val Ile Leu Gly Ser Pro Ala His Arg
            260                 265                 270

Ala Gly Leu Arg Pro Gly Asp Val Ile Leu Ala Ile Gly Glu Gln Met
        275                 280                 285

Val Gln Asn Ala Glu Asp Val Tyr Glu Ala Val Arg Thr Gln Ser Gln
    290                 295                 300

Leu Ala Val Gln Ile Arg Arg Gly Arg Glu Thr Leu Thr Leu Tyr Val
305                 310                 315                 320

Thr Pro Glu Val Thr Glu
                325

<210>  7
<211>  969
<212>  DNA
<213>  Artificial

<220>
<223>  Polynucleotide consisting of the same base sequence of SEQ ID NO:
       3 wherein the nucleotides of position 4-15 are deleted

<220>
<221>  misc_feature
<223>  DNA that codes for mature HtrA2(delta AVPS)

<400>  7
atgccgccgc ccgcttctcc ccggagtcag tacaacttca tcgcagatgt ggtggagaag      60

acagcacctg ccgtggtcta tatcgagatc ctggaccggc accctttctt gggccgcgag     120

gtccctatct cgaacggctc aggattcgtg gtggctgccg atgggctcat tgtcaccaac     180

gcccatgtgg tggctgatcg gcgcagagtc cgtgtgagac tgctaagcgg cgacacgtat     240

gaggccgtgg tcacagctgt ggatcccgtg gcagacatcg caacgctgag gattcagact     300

aaggagcctc tccccacgct gcctctggga cgctcagctg atgtccggca aggggagttt     360

gttgttgcca tgggaagtcc ctttgcactg cagaacacga tcacatccgg cattgttagc     420

tctgctcagc gtccagccag agacctggga ctcccccaaa ccaatgtgga atacattcaa     480

actgatgcag ctattgattt tggaaactct ggaggtcccc tggttaacct ggatggggag     540

gtgattggag tgaacaccat gaaggtcaca gctggaatct cctttgccat cccttctgat     600

cgtcttcgag agtttctgca tcgtggggaa aagaagaatt cctcctccgg aatcagtggg 660

tcccagcggc gctacattgg ggtgatgatg ctgaccctga gtcccagcat ccttgctgaa 720

ctacagcttc gagaaccaag ctttcccgat gttcagcatg gtgtactcat ccataaagtc 780

atcctgggct cccctgcaca ccgggctggt ctgcggcctg gtgatgtgat tttggccatt 840

ggggagcaga tggtacaaaa tgctgaagat gtttatgaag ctgttcgaac ccaatcccag 900

ttggcagtgc agatccggcg gggacgagaa acactgacct tatatgtgac ccctgaggtc 960

acagaatga 969

<210> 8
<211> 322
<212> PRT
<213> Artificial

<220>
<223> Polypeptide consisting of the same amino acid sequence of SEQ ID
NO:4 wherein the amino acid residues from the 2nd to the 5th are
deleted

<220>
<221> misc_feature
<223> mature HtrA2(delta AVPS)

<400> 8

Met Pro Pro Pro Ala Ser Pro Arg Ser Gln Tyr Asn Phe Ile Ala Asp
1               5                   10                  15

Val Val Glu Lys Thr Ala Pro Ala Val Val Tyr Ile Glu Ile Leu Asp
            20                  25                  30

Arg His Pro Phe Leu Gly Arg Glu Val Pro Ile Ser Asn Gly Ser Gly
        35                  40                  45

Phe Val Val Ala Ala Asp Gly Leu Ile Val Thr Asn Ala His Val Val
    50                  55                  60

Ala Asp Arg Arg Arg Val Arg Val Arg Leu Leu Ser Gly Asp Thr Tyr
65                  70                  75                  80

Glu Ala Val Val Thr Ala Val Asp Pro Val Ala Asp Ile Ala Thr Leu
                85                  90                  95

Arg Ile Gln Thr Lys Glu Pro Leu Pro Thr Leu Pro Leu Gly Arg Ser
            100                 105                 110

Ala Asp Val Arg Gln Gly Glu Phe Val Val Ala Met Gly Ser Pro Phe
        115                 120                 125

Ala Leu Gln Asn Thr Ile Thr Ser Gly Ile Val Ser Ser Ala Gln Arg
    130                 135                 140

Pro Ala Arg Asp Leu Gly Leu Pro Gln Thr Asn Val Glu Tyr Ile Gln
145                 150                 155                 160

Thr Asp Ala Ala Ile Asp Phe Gly Asn Ser Gly Gly Pro Leu Val Asn
                165                 170                 175

Leu Asp Gly Glu Val Ile Gly Val Asn Thr Met Lys Val Thr Ala Gly
            180                 185                 190

Ile Ser Phe Ala Ile Pro Ser Asp Arg Leu Arg Glu Phe Leu His Arg
        195                 200                 205

Gly Glu Lys Lys Asn Ser Ser Ser Gly Ile Ser Gly Ser Gln Arg Arg
    210                 215                 220

Tyr Ile Gly Val Met Met Leu Thr Leu Ser Pro Ser Ile Leu Ala Glu
225                 230                 235                 240

Leu Gln Leu Arg Glu Pro Ser Phe Pro Asp Val Gln His Gly Val Leu
                245                 250                 255

Ile His Lys Val Ile Leu Gly Ser Pro Ala His Arg Ala Gly Leu Arg
            260                 265                 270

Pro Gly Asp Val Ile Leu Ala Ile Gly Glu Gln Met Val Gln Asn Ala
        275                 280                 285

Glu Asp Val Tyr Glu Ala Val Arg Thr Gln Ser Gln Leu Ala Val Gln
    290                 295                 300

Ile Arg Arg Gly Arg Glu Thr Leu Thr Leu Tyr Val Thr Pro Glu Val
305                 310                 315                 320

Thr Glu

<210>  9
<211>  981
<212>  DNA
<213>  Artificial

<220>
<223>  Polynucleotide consisting of the same base sequence of SEQ ID NO:
       3 wherein the nucleotide of position 5 is g

<220>
<221>  misc_feature
<223>  DNA that codes for mature HtrA2(GVPS)

<400>  9
atgggcgtcc ctagcccgcc gcccgcttct ccccggagtc agtacaactt catcgcagat      60

gtggtggaga agacagcacc tgccgtggtc tatatcgaga tcctggaccg gcaccctttc     120

ttgggccgcg aggtccctat ctcgaacggc tcaggattcg tggtggctgc cgatgggctc     180

attgtcacca acgcccatgt ggtggctgat cggcgcagag tccgtgtgag actgctaagc     240

ggcgacacgt atgaggccgt ggtcacagct gtggatcccg tggcagacat cgcaacgctg     300

aggattcaga ctaaggagcc tctccccacg ctgcctctgg gacgctcagc tgatgtccgg     360

caaggggagt ttgttgttgc catgggaagt ccctttgcac tgcagaacac gatcacatcc     420

ggcattgtta gctctgctca gcgtccagcc agagacctgg gactccccca aaccaatgtg     480

gaatacattc aaactgatgc agctattgat tttggaaact ctggaggtcc cctggttaac     540

ctggatgggg aggtgattgg agtgaacacc atgaaggtca cagctggaat ctcctttgcc     600

atcccttctg atcgtcttcg agagtttctg catcgtgggg aaaagaagaa ttcctcctcc 660

ggaatcagtg ggtcccagcg gcgctacatt ggggtgatga tgctgaccct gagtcccagc 720

atccttgctg aactacagct tcgagaacca agctttcccg atgttcagca tggtgtactc 780

atccataaag tcatcctggg ctcccctgca caccgggctg gtctgcggcc tggtgatgtg 840

attttggcca ttggggagca gatggtacaa aatgctgaag atgtttatga agctgttcga 900

acccaatccc agttggcagt gcagatccgg cggggacgag aaacactgac cttatatgtg 960

acccctgagg tcacagaatg a 981

<210> 10
<211> 326
<212> PRT
<213> Artificial

<220>
<223> Polypeptide consisting of the same amino acid sequence of SEQ ID
NO:4 wherein the 2nd amino acid residue is substituted by Gly

<220>
<221> misc_feature
<223> mature HtrA2(GVPS)

<400> 10

Met Gly Val Pro Ser Pro Pro Pro Ala Ser Pro Arg Ser Gln Tyr Asn
1               5                   10                  15

Phe Ile Ala Asp Val Val Glu Lys Thr Ala Pro Ala Val Val Tyr Ile
            20                  25                  30

Glu Ile Leu Asp Arg His Pro Phe Leu Gly Arg Glu Val Pro Ile Ser
        35                  40                  45

Asn Gly Ser Gly Phe Val Val Ala Ala Asp Gly Leu Ile Val Thr Asn
    50                  55                  60

Ala His Val Val Ala Asp Arg Arg Arg Val Arg Val Arg Leu Leu Ser
65                  70                  75                  80

Gly Asp Thr Tyr Glu Ala Val Val Thr Ala Val Asp Pro Val Ala Asp
                85                  90                  95

Ile Ala Thr Leu Arg Ile Gln Thr Lys Glu Pro Leu Pro Thr Leu Pro
            100                 105                 110

Leu Gly Arg Ser Ala Asp Val Arg Gln Gly Glu Phe Val Val Ala Met
        115                 120                 125

Gly Ser Pro Phe Ala Leu Gln Asn Thr Ile Thr Ser Gly Ile Val Ser
    130                 135                 140

Ser Ala Gln Arg Pro Ala Arg Asp Leu Gly Leu Pro Gln Thr Asn Val
145                 150                 155                 160

Glu Tyr Ile Gln Thr Asp Ala Ala Ile Asp Phe Gly Asn Ser Gly Gly
                165                 170                 175

Pro Leu Val Asn Leu Asp Gly Glu Val Ile Gly Val Asn Thr Met Lys

180                 185                 190

Val Thr Ala Gly Ile Ser Phe Ala Ile Pro Ser Asp Arg Leu Arg Glu
        195                 200                 205

Phe Leu His Arg Gly Glu Lys Lys Asn Ser Ser Ser Gly Ile Ser Gly
    210                 215                 220

Ser Gln Arg Arg Tyr Ile Gly Val Met Met Leu Thr Leu Ser Pro Ser
225                 230                 235                 240

Ile Leu Ala Glu Leu Gln Leu Arg Glu Pro Ser Phe Pro Asp Val Gln
                245                 250                 255

His Gly Val Leu Ile His Lys Val Ile Leu Gly Ser Pro Ala His Arg
            260                 265                 270

Ala Gly Leu Arg Pro Gly Asp Val Ile Leu Ala Ile Gly Glu Gln Met
        275                 280                 285

Val Gln Asn Ala Glu Asp Val Tyr Glu Ala Val Arg Thr Gln Ser Gln
    290                 295                 300

Leu Ala Val Gln Ile Arg Arg Gly Arg Glu Thr Leu Thr Leu Tyr Val
305                 310                 315                 320

Thr Pro Glu Val Thr Glu
                325

<210>  11
<211>  969
<212>  DNA
<213>  Artificial

<220>
<223>  Polynucleotide consisting of the same base sequence of SEQ ID NO:
       5 wherein the nucleotides of position 4-15 are deleted

<220>
<221>  misc_feature
<223>  DNA that codes for mature HtrA2(S306, delta AVPS)

<400>  11
atgccgccgc ccgcttctcc ccggagtcag tacaacttca tcgcagatgt ggtggagaag      60

acagcacctg ccgtggtcta tatcgagatc ctggaccggc accctttctt gggccgcgag     120

gtccctatct cgaacggctc aggattcgtg gtggctgccg atgggctcat tgtcaccaac     180

gcccatgtgg tggctgatcg gcgcagagtc cgtgtgagac tgctaagcgg cgacacgtat     240

gaggccgtgg tcacagctgt ggatcccgtg gcagacatcg caacgctgag gattcagact     300

aaggagcctc tccccacgct gcctctggga cgctcagctg atgtccggca aggggagttt     360

gttgttgcca tgggaagtcc ctttgcactg cagaacacga tcacatccgg cattgttagc     420

tctgctcagc gtccagccag agacctggga ctcccccaaa ccaatgtgga atacattcaa     480

actgatgcag ctattgattt tggaaacgct ggaggtcccc tggttaacct ggatggggag     540

gtgattggag tgaacaccat gaaggtcaca gctggaatct cctttgccat cccttctgat     600

cgtcttcgag agtttctgca tcgtggggaa aagaagaatt cctcctccgg aatcagtggg     660

tcccagcggc gctacattgg ggtgatgatg ctgaccctga gtcccagcat ccttgctgaa 720

ctacagcttc gagaaccaag ctttcccgat gttcagcatg gtgtactcat ccataaagtc 780

atcctgggct cccctgcaca ccgggctggt ctgcggcctg gtgatgtgat tttggccatt 840

ggggagcaga tggtacaaaa tgctgaagat gtttatgaag ctgttcgaac ccaatcccag 900

ttggcagtgc agatccggcg gggacgagaa acactgacct tatatgtgac ccctgaggtc 960

acagaatga 969

<210> 12
<211> 322
<212> PRT
<213> Artificial

<220>
<223> Polypeptide consisting of the same amino acid sequence of SEQ ID
NO:6 wherein the amino acid residues from the 2nd to the 5th are
deleted

<220>
<221> misc_feature
<223> mature HtrA2(S306A, delta AVPS)

<400> 12

Met Pro Pro Pro Ala Ser Pro Arg Ser Gln Tyr Asn Phe Ile Ala Asp
1               5                   10                  15

Val Val Glu Lys Thr Ala Pro Ala Val Val Tyr Ile Glu Ile Leu Asp
            20                  25                  30

Arg His Pro Phe Leu Gly Arg Glu Val Pro Ile Ser Asn Gly Ser Gly
        35                  40                  45

Phe Val Val Ala Ala Asp Gly Leu Ile Val Thr Asn Ala His Val Val
    50                  55                  60

Ala Asp Arg Arg Arg Val Arg Val Arg Leu Leu Ser Gly Asp Thr Tyr
65                  70                  75                  80

Glu Ala Val Val Thr Ala Val Asp Pro Val Ala Asp Ile Ala Thr Leu
                85                  90                  95

Arg Ile Gln Thr Lys Glu Pro Leu Pro Thr Leu Pro Leu Gly Arg Ser
            100                 105                 110

Ala Asp Val Arg Gln Gly Glu Phe Val Val Ala Met Gly Ser Pro Phe
        115                 120                 125

Ala Leu Gln Asn Thr Ile Thr Ser Gly Ile Val Ser Ser Ala Gln Arg
    130                 135                 140

Pro Ala Arg Asp Leu Gly Leu Pro Gln Thr Asn Val Glu Tyr Ile Gln
145                 150                 155                 160

Thr Asp Ala Ala Ile Asp Phe Gly Asn Ala Gly Gly Pro Leu Val Asn
                165                 170                 175

Leu Asp Gly Glu Val Ile Gly Val Asn Thr Met Lys Val Thr Ala Gly
            180                 185                 190

Ile Ser Phe Ala Ile Pro Ser Asp Arg Leu Arg Glu Phe Leu His Arg
        195                 200                 205

Gly Glu Lys Lys Asn Ser Ser Ser Gly Ile Ser Gly Ser Gln Arg Arg
    210                 215                 220

Tyr Ile Gly Val Met Met Leu Thr Leu Ser Pro Ser Ile Leu Ala Glu
225                 230                 235                 240

Leu Gln Leu Arg Glu Pro Ser Phe Pro Asp Val Gln His Gly Val Leu
                245                 250                 255

Ile His Lys Val Ile Leu Gly Ser Pro Ala His Arg Ala Gly Leu Arg
            260                 265                 270

Pro Gly Asp Val Ile Leu Ala Ile Gly Glu Gln Met Val Gln Asn Ala
        275                 280                 285

Glu Asp Val Tyr Glu Ala Val Arg Thr Gln Ser Gln Leu Ala Val Gln
    290                 295                 300

Ile Arg Arg Gly Arg Glu Thr Leu Thr Leu Tyr Val Thr Pro Glu Val
305                 310                 315                 320

Thr Glu

<210>  13
<211>  981
<212>  DNA
<213>  Artificial

<220>
<223>  Polynucleotide consisting of the same base sequence of SEQ ID NO:
       5 wherein the nucleotide of position 5 is g

<220>
<221>  misc_feature
<223>  DNA that codes for mature HtrA2(S306A, GVPS)

<400>  13
atgggcgtcc ctagcccgcc gcccgcttct ccccggagtc agtacaactt catcgcagat      60

gtggtggaga agacagcacc tgccgtggtc tatatcgaga tcctggaccg gcaccctttc     120

ttgggccgcg aggtccctat ctcgaacggc tcaggattcg tggtggctgc cgatgggctc     180

attgtcacca acgcccatgt ggtggctgat cggcgcagag tccgtgtgag actgctaagc     240

ggcgacacgt atgaggccgt ggtcacagct gtggatcccg tggcagacat cgcaacgctg     300

aggattcaga ctaaggagcc tctccccacg ctgcctctgg gacgctcagc tgatgtccgg     360

caaggggagt ttgttgttgc catgggaagt ccctttgcac tgcagaacac gatcacatcc     420

ggcattgtta gctctgctca gcgtccagcc agagacctgg gactccccca aaccaatgtg     480

gaatacattc aaactgatgc agctattgat tttggaaacg ctggaggtcc cctggttaac     540

ctggatgggg aggtgattgg agtgaacacc atgaaggtca cagctggaat ctcctttgcc     600

atcccttctg atcgtcttcg agagtttctg catcgtgggg aaaagaagaa ttcctcctcc     660

ggaatcagtg ggtcccagcg gcgctacatt ggggtgatga tgctgaccct gagtcccagc     720

atccttgctg aactacagct tcgagaacca agctttcccg atgttcagca tggtgtactc 780

atccataaag tcatcctggg ctcccctgca caccgggctg gtctgcggcc tggtgatgtg 840

attttggcca ttggggagca gatggtacaa aatgctgaag atgtttatga agctgttcga 900

acccaatccc agttggcagt gcagatccgg cggggacgag aaacactgac cttatatgtg 960

acccctgagg tcacagaatg a 981

<210> 14
<211> 326
<212> PRT
<213> Artificial

<220>
<223> Polypeptide consisting of the same amino acid sequence of SEQ ID
NO:6 wherein the 2nd amino acid residue is substituted by Gly

<220>
<221> misc_feature
<223> mature HtrA2(S306A, GVPS)

<400> 14

Met Gly Val Pro Ser Pro Pro Pro Ala Ser Pro Arg Ser Gln Tyr Asn
1               5                   10                  15

Phe Ile Ala Asp Val Val Glu Lys Thr Ala Pro Ala Val Val Tyr Ile
            20                  25                  30

Glu Ile Leu Asp Arg His Pro Phe Leu Gly Arg Glu Val Pro Ile Ser
        35                  40                  45

Asn Gly Ser Gly Phe Val Val Ala Ala Asp Gly Leu Ile Val Thr Asn
    50                  55                  60

Ala His Val Val Ala Asp Arg Arg Arg Val Arg Val Arg Leu Leu Ser
65                  70                  75                  80

Gly Asp Thr Tyr Glu Ala Val Val Thr Ala Val Asp Pro Val Ala Asp
                85                  90                  95

Ile Ala Thr Leu Arg Ile Gln Thr Lys Glu Pro Leu Pro Thr Leu Pro
            100                 105                 110

Leu Gly Arg Ser Ala Asp Val Arg Gln Gly Glu Phe Val Val Ala Met
        115                 120                 125

Gly Ser Pro Phe Ala Leu Gln Asn Thr Ile Thr Ser Gly Ile Val Ser
    130                 135                 140

Ser Ala Gln Arg Pro Ala Arg Asp Leu Gly Leu Pro Gln Thr Asn Val
145                 150                 155                 160

Glu Tyr Ile Gln Thr Asp Ala Ala Ile Asp Phe Gly Asn Ala Gly Gly
                165                 170                 175

Pro Leu Val Asn Leu Asp Gly Glu Val Ile Gly Val Asn Thr Met Lys
            180                 185                 190

Val Thr Ala Gly Ile Ser Phe Ala Ile Pro Ser Asp Arg Leu Arg Glu
        195                 200                 205

Phe Leu His Arg Gly Glu Lys Lys Asn Ser Ser Ser Gly Ile Ser Gly
    210                 215                 220

Ser Gln Arg Arg Tyr Ile Gly Val Met Met Leu Thr Leu Ser Pro Ser
225                 230                 235                 240

Ile Leu Ala Glu Leu Gln Leu Arg Glu Pro Ser Phe Pro Asp Val Gln
                245                 250                 255

His Gly Val Leu Ile His Lys Val Ile Leu Gly Ser Pro Ala His Arg
            260                 265                 270

Ala Gly Leu Arg Pro Gly Asp Val Ile Leu Ala Ile Gly Glu Gln Met
        275                 280                 285

Val Gln Asn Ala Glu Asp Val Tyr Glu Ala Val Arg Thr Gln Ser Gln
    290                 295                 300

Leu Ala Val Gln Ile Arg Arg Gly Arg Glu Thr Leu Thr Leu Tyr Val
305                 310                 315                 320

Thr Pro Glu Val Thr Glu
                325

<210>  15
<211>  1785
<212>  DNA
<213>  Homo sapiens

<220>
<221>  misc_feature
<223>  DNA that codes for SHC3

<400>  15
atgcttccac gcaccaagta taaccgcttc aggaatgact cggtgacatc ggtcgatgac      60

cttctccaca gcctgtcggt gagcggcggc ggaggcaagg tttcggcggc gcgcgcgacc     120

ccggcggcgg ctccctactt ggtgtccggc gaggcgctgc gcaaggcgcc cgacgatggg     180

cccggcagcc tgggccacct gctccacaag gtgtcccacc tgaaactctc cagctcgggc     240

ctccgcggcc tgtcgtcggc cgcccgggag cgggcgggcg cgcggctctc gggcagctgc     300

agcgcgccca gcctggccgc cccggacggc agtgcgccct cggcgccccg cgccccggcc     360

atgagcgccg ccaggaaggg ccggcccggc gacgagccgc tgcccaggcc ccctcggggg     420

gcgccgcacg ccagcgacca ggtgctgggg cccggagtca cctacgtggt caagtacttg     480

gggtgcattg aagttctgcg ctcaatgagg tctcttgact tcagtacaag aacacaaatt     540

accagggaag ccatcagccg cgtctgtgaa gctgtgcctg gtgcgaaggg agccttcaag     600

aagagaaagc ctccaagcaa aatgctgtcc agcatcttgg gaaagagcaa cctccagttt     660

gcgggaatga gcatctctct gaccatctcc acggccagtc tgaacctgcg aactccggac     720

tccaaacaga tcatagcgaa tcaccacatg cggtccatct ccttcgcctc tgggggagac     780

ccggacacaa ctgactatgt tgcatatgtg gctaaggacc ctgttaatcg cagagcttgt     840

cacattttgg aatgctgtga tgggctggcc caggatgtca tcggctccat cggacaagcc     900

tttgagctcc ggtttaagca atatttacag tgtcctacca agattcccgc tctccatgat 960

cgaatgcaga gtctggatga gccatggacg gaagaggagg gagatggctc agaccaccca 1020

tactacaaca gcatcccaag caagatgcct cctccagggg gctttcttga tactagactg 1080

aaacccagac cccatgctcc tgacacagcc cagtttgcag gaaaagagca gacttattac 1140

cagggaagac acttaggaga cacttttggc gaagactggc agcaaacacc tttaaggcaa 1200

gggtcctcgg acatctacag cacgccagaa gggaaactgc acgtggcccc cacgggagaa 1260

gcacccacct acgtcaacac tcagcagatc ccaccacagg cctggccggc tgcggtcagc 1320

agtgctgaga gcagcccaag gaaagacctc tttgacatga aaccttttga agatgctctc 1380

aagaaccagc ccttggggcc cgtgttaagc aaggcagcct ccgtggagtg catcagccct 1440

gtgtcaccta gagccccaga tgccaagatg ctggaggaac tgcaagccga gacttggtac 1500

caaggagaga tgagcaggaa ggaggcagag gggctgctgg agaaagacgg agacttcctg 1560

gtcaggaaga gcaccaccaa cccgggctcc tttgtcctca cgggcatgca caatggccag 1620

gccaagcacc tgctgctcgt ggacccagaa ggcacgatcc ggacaaagga cagagtcttt 1680

gacagtatca gccacctcat caaccaccac ctagaaagca gcctgcccat tgtctctgca 1740

gggagtgagc tgtgtctcca gcagccagtg gagaggaagc agtga 1785

<210> 16
<211> 594
<212> PRT
<213> Homo sapiens

<220>
<221> misc_feature
<223> SHC3

<400> 16

Met Leu Pro Arg Thr Lys Tyr Asn Arg Phe Arg Asn Asp Ser Val Thr
1               5                   10                  15

Ser Val Asp Asp Leu Leu His Ser Leu Ser Val Ser Gly Gly Gly Gly
            20                  25                  30

Lys Val Ser Ala Ala Arg Ala Thr Pro Ala Ala Ala Pro Tyr Leu Val
        35                  40                  45

Ser Gly Glu Ala Leu Arg Lys Ala Pro Asp Asp Gly Pro Gly Ser Leu
    50                  55                  60

Gly His Leu Leu His Lys Val Ser His Leu Lys Leu Ser Ser Ser Gly
65                  70                  75                  80

Leu Arg Gly Leu Ser Ser Ala Ala Arg Glu Arg Ala Gly Ala Arg Leu
                85                  90                  95

Ser Gly Ser Cys Ser Ala Pro Ser Leu Ala Ala Pro Asp Gly Ser Ala
            100                 105                 110

Pro Ser Ala Pro Arg Ala Pro Ala Met Ser Ala Ala Arg Lys Gly Arg
        115                 120                 125

Pro Gly Asp Glu Pro Leu Pro Arg Pro Pro Arg Gly Ala Pro His Ala
    130                 135                 140

Ser Asp Gln Val Leu Gly Pro Gly Val Thr Tyr Val Val Lys Tyr Leu
145                 150                 155                 160

Gly Cys Ile Glu Val Leu Arg Ser Met Arg Ser Leu Asp Phe Ser Thr
                165                 170                 175

Arg Thr Gln Ile Thr Arg Glu Ala Ile Ser Arg Val Cys Glu Ala Val
            180                 185                 190

Pro Gly Ala Lys Gly Ala Phe Lys Lys Arg Lys Pro Pro Ser Lys Met
        195                 200                 205

Leu Ser Ser Ile Leu Gly Lys Ser Asn Leu Gln Phe Ala Gly Met Ser
    210                 215                 220

Ile Ser Leu Thr Ile Ser Thr Ala Ser Leu Asn Leu Arg Thr Pro Asp
225                 230                 235                 240

Ser Lys Gln Ile Ile Ala Asn His His Met Arg Ser Ile Ser Phe Ala
                245                 250                 255

Ser Gly Gly Asp Pro Asp Thr Thr Asp Tyr Val Ala Tyr Val Ala Lys
            260                 265                 270

Asp Pro Val Asn Arg Arg Ala Cys His Ile Leu Glu Cys Cys Asp Gly
        275                 280                 285

Leu Ala Gln Asp Val Ile Gly Ser Ile Gly Gln Ala Phe Glu Leu Arg
    290                 295                 300

Phe Lys Gln Tyr Leu Gln Cys Pro Thr Lys Ile Pro Ala Leu His Asp
305                 310                 315                 320

Arg Met Gln Ser Leu Asp Glu Pro Trp Thr Glu Glu Glu Gly Asp Gly
                325                 330                 335

Ser Asp His Pro Tyr Tyr Asn Ser Ile Pro Ser Lys Met Pro Pro Pro
            340                 345                 350

Gly Gly Phe Leu Asp Thr Arg Leu Lys Pro Arg Pro His Ala Pro Asp
        355                 360                 365

Thr Ala Gln Phe Ala Gly Lys Glu Gln Thr Tyr Tyr Gln Gly Arg His
    370                 375                 380

Leu Gly Asp Thr Phe Gly Glu Asp Trp Gln Gln Thr Pro Leu Arg Gln
385                 390                 395                 400

Gly Ser Ser Asp Ile Tyr Ser Thr Pro Glu Gly Lys Leu His Val Ala
                405                 410                 415

Pro Thr Gly Glu Ala Pro Thr Tyr Val Asn Thr Gln Gln Ile Pro Pro
            420                 425                 430

Gln Ala Trp Pro Ala Ala Val Ser Ser Ala Glu Ser Ser Pro Arg Lys
        435                 440                 445

Asp Leu Phe Asp Met Lys Pro Phe Glu Asp Ala Leu Lys Asn Gln Pro
    450                 455                 460

Leu Gly Pro Val Leu Ser Lys Ala Ala Ser Val Glu Cys Ile Ser Pro
465                 470                 475                 480

Val Ser Pro Arg Ala Pro Asp Ala Lys Met Leu Glu Glu Leu Gln Ala
                485                 490                 495

Glu Thr Trp Tyr Gln Gly Glu Met Ser Arg Lys Glu Ala Glu Gly Leu
            500                 505                 510

Leu Glu Lys Asp Gly Asp Phe Leu Val Arg Lys Ser Thr Thr Asn Pro
        515                 520                 525

Gly Ser Phe Val Leu Thr Gly Met His Asn Gly Gln Ala Lys His Leu
    530                 535                 540

Leu Leu Val Asp Pro Glu Gly Thr Ile Arg Thr Lys Asp Arg Val Phe
545                 550                 555                 560

Asp Ser Ile Ser His Leu Ile Asn His His Leu Glu Ser Ser Leu Pro
                565                 570                 575

Ile Val Ser Ala Gly Ser Glu Leu Cys Leu Gln Gln Pro Val Glu Arg
            580                 585                 590

Lys Gln

<210>  17
<211>  2112
<212>  DNA
<213>  Homo sapiens

<220>
<221>  misc_feature
<223>  DNA that codes for CREBL1

<400>  17
atggcggagc tgatgctgct cagcgagatt gctgacccga cgcgtttctt caccgacaac      60

ctgcttagcc cggaggactg gggtctgcag aacagcacct tgtattctgg cctagatgaa      120

gtggccgagg agcagacgca gctcttccgt tgcccggagc aggatgtccc gtttgacggc      180

agctccctgg acgtggggat ggatgtcagc ccctctgagc ccccatggga actcctgccg      240

atcttcccag atcttcaggt gaagtctgag ccatcttccc cctgctcttc ctcctccctc      300

agctccgagt catcgcgtct ctccacagag ccatccagcg aggctcttgg ggtaggggag      360

gtgctccatg tgaagacaga gtccttggca cccccactgt gtctcctggg agatgaccca      420

acatcctcat ttgaaaccgt ccagatcaat gttatcccca cctctgatga ttcctcagat      480

gtccagacca agatagaacc tgtctctcca tgttcttccg tcaactctga ggcctccctg      540

ctctcagccg actcctccag ccaggctttt ataggagagg aggtcctgga agtgaagaca      600

gagtccctgt ccccttcagg atgcctcctg tgggatgtcc cagccccctc acttggagct      660

gtccagatca gcatgggccc atcccttgat ggctcctcag gcaaagccct gcccacccgg 720
aagccgccac tgcagcccaa acctgtagtg ctaaccactg tcccaatgcc atccagagct 780
gtgcctccca gcaccacagt ccttctgcag tccctcgtcc agccaccccc agtgtcccca 840
gttgtcctca tccagggtgc tattcgagtc cagcctgaag ggccggctcc ctctctacca 900
cggcctgaga ggaagagcat cgttcccgct cctatgcctg gaaactcctg cccgcctgaa 960
gtggatgcaa agctgctgaa gcggcagcag cgaatgatca agaaccggga gtcagcctgc 1020
cagtcccgga gaaagaagaa agagtatctg cagggactgg aggctcggct gcaagcagta 1080
ctggctgaca accagcagct ccgccgagag aatgctgccc tccggcggcg gctggaggcc 1140
ctgctggctg aaaacagcga gctcaagtta gggtctggaa acaggaaggt ggtctgcatc 1200
atggtcttcc ttctcttcat tgccttcaac tttggacctg tcagcatcag tgagcctcct 1260
tcagctccca tctctcctcg gatgaacaag ggggagcctc aaccccggag acacttgctg 1320
gggttctcag agcaagagcc agttcaggga gttgaacctc tccaggggtc ctcccagggc 1380
cctaaggagc cccagcccag ccccacagac cagcccagtt tcagcaacct gacagccttc 1440
cctgggggcg ccaaggagct actactaaga gacctagacc agctcttcct ctcctctgat 1500
tgccggcact tcaaccgcac tgagtccctg aggcttgctg acgagttgag tggctgggtc 1560
cagcgccacc agagaggccg gaggaagatc cctcagaggg cccaggagag acagaagtct 1620
cagccacgga agaagtcacc tccagttaag gcagtcccca tccaaccccc tggaccccca 1680
gaaagggatt ctgtggccca gctgcaacta tatcgccacc cagaccgttc gcagccagca 1740
ttcttggatg caattgaccg acgggaagac acattttatg ttgtctcttt ccgaagggac 1800
cacctgctgc tcccagccat cagccacaac aagacctccc ggcccaagat gtccctggtg 1860
atgcctgcca tggcccccaa tgagaccctg tcaggccgtg ggccccgggg ggactatgag 1920
gagatgatgc agatcgagtg tgaggtcatg gacaccaggg tgattcacat caagacctcc 1980
acagtgcccc cctcgctccg aaaacagcca tccccaaccc caggcaatgc cacaggtggc 2040
cccttgccag tctctgcagc cagccaggcc caccaggcct cccaccagcc cctctacctc 2100
aatcatccct aa 2112

<210> 18
<211> 703
<212> PRT
<213> Homo sapiens

<220>
<221> misc_feature
<223> CREBL1

<400> 18

Met Ala Glu Leu Met Leu Leu Ser Glu Ile Ala Asp Pro Thr Arg Phe
1               5                   10                  15

Phe Thr Asp Asn Leu Leu Ser Pro Glu Asp Trp Gly Leu Gln Asn Ser
            20                  25                  30

Thr Leu Tyr Ser Gly Leu Asp Glu Val Ala Glu Glu Gln Thr Gln Leu
        35                  40                  45

Phe Arg Cys Pro Glu Gln Asp Val Pro Phe Asp Gly Ser Ser Leu Asp
    50                  55                  60

Val Gly Met Asp Val Ser Pro Ser Glu Pro Pro Trp Glu Leu Leu Pro
65                  70                  75                  80

Ile Phe Pro Asp Leu Gln Val Lys Ser Glu Pro Ser Ser Pro Cys Ser
                85                  90                  95

Ser Ser Ser Leu Ser Ser Glu Ser Ser Arg Leu Ser Thr Glu Pro Ser
            100                 105                 110

Ser Glu Ala Leu Gly Val Gly Glu Val Leu His Val Lys Thr Glu Ser
        115                 120                 125

Leu Ala Pro Pro Leu Cys Leu Leu Gly Asp Asp Pro Thr Ser Ser Phe
    130                 135                 140

Glu Thr Val Gln Ile Asn Val Ile Pro Thr Ser Asp Asp Ser Ser Asp
145                 150                 155                 160

Val Gln Thr Lys Ile Glu Pro Val Ser Pro Cys Ser Ser Val Asn Ser
                165                 170                 175

Glu Ala Ser Leu Leu Ser Ala Asp Ser Ser Ser Gln Ala Phe Ile Gly
            180                 185                 190

Glu Glu Val Leu Glu Val Lys Thr Glu Ser Leu Ser Pro Ser Gly Cys
        195                 200                 205

Leu Leu Trp Asp Val Pro Ala Pro Ser Leu Gly Ala Val Gln Ile Ser
    210                 215                 220

Met Gly Pro Ser Leu Asp Gly Ser Ser Gly Lys Ala Leu Pro Thr Arg
225                 230                 235                 240

Lys Pro Pro Leu Gln Pro Lys Pro Val Val Leu Thr Thr Val Pro Met
                245                 250                 255

Pro Ser Arg Ala Val Pro Pro Ser Thr Thr Val Leu Leu Gln Ser Leu
            260                 265                 270

Val Gln Pro Pro Pro Val Ser Pro Val Val Leu Ile Gln Gly Ala Ile
        275                 280                 285

Arg Val Gln Pro Glu Gly Pro Ala Pro Ser Leu Pro Arg Pro Glu Arg
    290                 295                 300

Lys Ser Ile Val Pro Ala Pro Met Pro Gly Asn Ser Cys Pro Pro Glu
305                 310                 315                 320

Val Asp Ala Lys Leu Leu Lys Arg Gln Gln Arg Met Ile Lys Asn Arg
                325                 330                 335

Glu Ser Ala Cys Gln Ser Arg Arg Lys Lys Lys Glu Tyr Leu Gln Gly
            340                 345                 350

Leu Glu Ala Arg Leu Gln Ala Val Leu Ala Asp Asn Gln Gln Leu Arg
        355                 360                 365

Arg Glu Asn Ala Ala Leu Arg Arg Arg Leu Glu Ala Leu Leu Ala Glu
    370                 375                 380

Asn Ser Glu Leu Lys Leu Gly Ser Gly Asn Arg Lys Val Val Cys Ile
385                 390                 395                 400

Met Val Phe Leu Leu Phe Ile Ala Phe Asn Phe Gly Pro Val Ser Ile
                405                 410                 415

Ser Glu Pro Pro Ser Ala Pro Ile Ser Pro Arg Met Asn Lys Gly Glu
            420                 425                 430

Pro Gln Pro Arg Arg His Leu Leu Gly Phe Ser Glu Gln Glu Pro Val
        435                 440                 445

Gln Gly Val Glu Pro Leu Gln Gly Ser Ser Gln Gly Pro Lys Glu Pro
    450                 455                 460

Gln Pro Ser Pro Thr Asp Gln Pro Ser Phe Ser Asn Leu Thr Ala Phe
465                 470                 475                 480

Pro Gly Gly Ala Lys Glu Leu Leu Leu Arg Asp Leu Asp Gln Leu Phe
                485                 490                 495

Leu Ser Ser Asp Cys Arg His Phe Asn Arg Thr Glu Ser Leu Arg Leu
            500                 505                 510

Ala Asp Glu Leu Ser Gly Trp Val Gln Arg His Gln Arg Gly Arg Arg
        515                 520                 525

Lys Ile Pro Gln Arg Ala Gln Glu Arg Gln Lys Ser Gln Pro Arg Lys
    530                 535                 540

Lys Ser Pro Pro Val Lys Ala Val Pro Ile Gln Pro Pro Gly Pro Pro
545                 550                 555                 560

Glu Arg Asp Ser Val Gly Gln Leu Gln Leu Tyr Arg His Pro Asp Arg
                565                 570                 575

Ser Gln Pro Ala Phe Leu Asp Ala Ile Asp Arg Arg Glu Asp Thr Phe
            580                 585                 590

Tyr Val Val Ser Phe Arg Arg Asp His Leu Leu Leu Pro Ala Ile Ser
        595                 600                 605

His Asn Lys Thr Ser Arg Pro Lys Met Ser Leu Val Met Pro Ala Met
    610                 615                 620

Ala Pro Asn Glu Thr Leu Ser Gly Arg Gly Ala Pro Gly Asp Tyr Glu
625                 630                 635                 640

Glu Met Met Gln Ile Glu Cys Glu Val Met Asp Thr Arg Val Ile His
                645                 650                 655

Ile Lys Thr Ser Thr Val Pro Pro Ser Leu Arg Lys Gln Pro Ser Pro
            660                 665                 670

Thr Pro Gly Asn Ala Thr Gly Gly Pro Leu Pro Val Ser Ala Ala Ser
        675                 680                 685

Gln Ala His Gln Ala Ser His Gln Pro Leu Tyr Leu Asn His Pro
    690                 695                 700

<210>  19
<211>  2013
<212>  DNA
<213>  Homo sapiens

<220>
<221>  misc_feature
<223>  DNA that codes for ATF6

<400>  19
atgggggagc cggctggggt tgccggcacc atggagtcac cttttagccc gggactcttt      60
cacaggctgg atgaagattg ggattctgct ctctttgctg aactcggtta tttcacagac     120
actgatgagc tgcaattgga agcagcaaat gagacgtatg aaaacaattt tgataatctt     180
gattttgatt tggatttgat gccttgggag tcagacattt gggacatcaa caaccaaatc     240
tgtacagtta aagatattaa ggcagaacct cagccacttt ctccagcctc ctcaagttat     300
tcagtctcgt ctcctcggtc agtggactct tattcttcaa ctcagcatgt tcctgaggag     360
ttggatttgt cttctagttc tcagatgtct cccctttcct tatatggtga aaactctaat     420
agtctctctt cagcggagcc actgaaggaa gataagcctg tcactggtcc taggaacaag     480
actgaaaatg gactgactcc aaagaaaaaa attcaggtga attcaaaacc ttcaattcag     540
cccaagcctt tattgcttcc agcagcaccc aagactcaaa caaactccag tgttccagca     600
aaaaccatca ttattcagac agtaccaacg cttatgccat tggcaaagca gcaaccaatt     660
atcagtttac aacctgcacc cactaaaggc cagacggttt tgctgtctca gcctactgtg     720
gtacaacttc aagcacctgg agttctgccc tctgctcagc cagtccttgc tgttgctggg     780
ggagtcacac agctccctaa tcacgtggtg aatgtggtac cagccccttc agcgaatagc     840
ccagtgaatg gaaaactttc cgtgactaaa cctgtcctac aaagtaccat gagaaatgtc     900
ggttcagata ttgctgtgct aaggagacag caacgtatga taaaaaatcg agaatccgct     960
tgtcagtctc gcaagaagaa gaaagaatat atgctagggt tagaggcgag attaaaggct    1020
gccctctcag aaaacgagca actgaagaaa gaaaatggaa cactgaagcg gcagctggat    1080
gaagttgtgt cagagaacca gaggcttaaa gtccctagtc caaagcgaag agttgtctgt    1140
gtgatgatag tattggcatt tataatactg aactatggac ctatgagcat gttggaacag    1200
gattccagga gaatgaaccc tagtgtgagc cctgcaaatc aaaggaggca ccttctagga    1260
ttttctgcta aagaggcaca ggacacatca gatggtatta tccagaaaaa cagctacaga    1320
tatgatcatt ctgtttcaaa tgacaaagcc ctgatggtgc taactgaaga accattgctt    1380
tacattcctc cacctccttg tcagccccta attaacacaa cagagtctct caggttaaat    1440
catgaacttc gaggatgggt tcatagacat gaagtagaaa ggaccaagtc aagaagaatg    1500
acaaataatc aacagaaaac ccgtattctt cagggtgctc tggaacaggg ctcaaattct    1560
cagctgatgg ctgttcaata cacagaaacc actagtagta tcagcaggaa ctcagggagt    1620

gagctacaag tgtattatgc ttcacccaga agttatcaag acttttttga agccatccgc 1680

agaaggggag acacatttta tgttgtgtca tttcgaaggg atcacctgct gttaccagct 1740

accacccata acaagaccac aagaccaaaa atgtcaattg tgttaccagc aataaacata 1800

aatgagaatg tgatcaatgg gcaggactac gaagtgatga tgcagattga ctgtcaggtg 1860

atggacacca ggatcctcca tatcaaaagt tcgtcagttc ctccttacct ccgagatcag 1920

cagaggaatc aaaccaacac cttctttggc tcccctcccg cagccacaga ggcaacccac 1980

gttgtcagca ccatccctga gtcattacaa tag 2013

<210> 20
<211> 670
<212> PRT
<213> Homo sapiens

<220>
<221> misc_feature
<223> ATF6

<400> 20

<210>  21
<211>  36
<212>  DNA
<213>  Artificial

<220>
<223>  Designed polynucleotide based on the base sequence of SEQ ID NO:3
        for use as a primer to obtain mature HtrA2 DNA

<400>  21
catatggccg tccctagccc gccgcccgct tctccc                                36

<210>  22
<211>  35
<212>  DNA
<213>  Artificial

<220>
<223>  Designed polynucleotide based on the base sequence of SEQ ID NO:3
        for use as a primer to obtain mature HtrA2 DNA

<400>  22
ctcgagttct gtgacctcag gggtcacata taagg                                 35

<210>  23
<211>  40
<212>  DNA
<213>  Artificial

<220>

<223>  Designed polynucleotide based on the base sequence of SEQ ID NO:3
       for use as a primer to obtain mature HtrA2(S306A) DNA

<400>  23
gctattgatt ttggaaacgc tggaggtccc ctggttaacc                              40


<210>  24
<211>  40
<212>  DNA
<213>  Artificial

<220>
<223>  Designed polynucleotide based on the base sequence of SEQ ID NO:3
       for use as a primer to obtain mature HtrA2(S306A) DNA

<400>  24
ggttaaccag gggacctcca gcgtttccaa aatcaatagc                              40


<210>  25
<211>  39
<212>  DNA
<213>  Artificial

<220>
<223>  Designed polynucleotide based on the base sequence of SEQ ID NO:1
       5 for use as a primer to obtain SHC3 DNA

<400>  25
ggatccgcca tgcttccacg caccaagtat aaccgcttc                               39


<210>  26
<211>  37
<212>  DNA
<213>  Artificial

<220>
<223>  Designed polynucleotide based on the base sequence of SEQ ID NO:1
       5 for use as a primer to obtain SHC3 DNA

<400>  26
ctcgagctgc ttcctctcca ctggctgctg gagacac                                 37


<210>  27
<211>  27
<212>  DNA
<213>  Artificial

<220>
<223>  Designed polynucleotide based on the base sequence of SEQ ID NO:1
       7 for use as a primer to obtain CREBL1 DNA

<400>  27
gcgaattcgc catggcggag ctgatgc                                            27


<210>  28
<211>  28
<212>  DNA
<213>  Artificial

<220>
<223>  Designed polynucleotide based on the base sequence of SEQ ID NO:1
       7 for use as a primer to obtain CREBL1 DNA

<400>  28
gcctcgaggg gatgattgag gtagaggg                                           28


<210>  29
<211>  30
<212>  DNA
<213>  Artificial

<220>
<223>  Designed polynucleotide based on the base sequence of SEQ ID NO:1
      7 for use as a primer to obtain CREBL1 DNA

<400>  29
gcggatcccg cggagctgat gctgctcagc                                        30


<210>  30
<211>  33
<212>  DNA
<213>  Artificial

<220>
<223>  Designed polynucleotide based on the base sequence of SEQ ID NO:1
      7 for use as a primer to obtain CREBL1 DNA

<400>  30
cctcgaggtt tagggatgat tgaggtagag ggg                                    33


<210>  31
<211>  30
<212>  DNA
<213>  Artificial

<220>
<223>  Designed polynucleotide based on the base sequence of SEQ ID NO:1
      9 for use as a primer to obtain ATF6 DNA

<400>  31
agttccaggg aaaaggaact tgtgaaatgg                                        30


<210>  32
<211>  30
<212>  DNA
<213>  Artificial

<220>
<223>  Designed polynucleotide based on the base sequence of SEQ ID NO:1
      9 for use as a primer to obtain ATF6 DNA

<400>  32
acgctcagtt ttccacatag ctgcgggtgc                                        30


<210>  33
<211>  39
<212>  DNA
<213>  Artificial

<220>
<223>  Designed polynucleotide based on the base sequence of SEQ ID NO:1
      9 for use as a primer to obtain ATF6 DNA

<400>  33
aaagatatca tgggggagcc ggctggggtt gccggcacc                              39


<210>  34
<211>  39
<212>  DNA
<213>  Artificial

<220>
<223>  Designed polynucleotide based on the base sequence of SEQ ID NO:1
      9 for use as a primer to obtain ATF6 DNA

<400>  34
aaactcgagc tattgtaatg actcagggat ggtgctgac                              39


<210>  35
<211>  39

<212>  DNA
<213>  Artificial

<220>
<223>  Designed polynucleotide based on the base sequence of SEQ ID NO:1
       9 for use as a primer to obtain ATF6 DNA

<400>  35
aaaagatcta tgggggagcc ggctggggtt gccggcacc                          39


<210>  36
<211>  36
<212>  DNA
<213>  Artificial

<220>
<223>  Designed polynucleotide based on the base sequence of SEQ ID NO:3
        for use as a primer to obtain mature HtrA2(delta AVPS) DNA

<400>  36
gagctcatgc cgccgcccgc ttctccccgg agtcag                             36


<210>  37
<211>  36
<212>  DNA
<213>  Artificial

<220>
<223>  Designed polynucleotide based on the base sequence of SEQ ID NO:3
        for use as a primer to obtain mature HtrA2(GVPS) DNA

<400>  37
gagctcatgg gcgtccctag cccgccgccc gcttct                             36